# EUROPÄISCHE PATENTSCHRIFT

(11) **EP 3 166 584 B1**
(45) Veröffentlichungstag und Bekanntmachung des Hinweises auf die Patenterteilung: **28.11.2018**
(21) Anmeldenummer: 15728532.1
(22) Anmeldetag: 12.06.2015
(51) Int. Cl.: A61K 8/44, A61K 8/45, A61K 8/46, A61Q 5/08

(54) **VERBESSERTE ENTFÄRBUNG VON GEFÄRBTEN KERATINISCHEN FASERN**
IMPROVED DECOLOURIZATION OF COLOURED KERATINIC FIBRES
DÉCOLORATION AMÉLIORÉE DE FIBRES KÉRATINIQUES COLORÉES

(30) Priorität: 09.07.2014 DE 102014213317
(43) Veröffentlichungstag der Anmeldung: 17.05.2017
(73) Patentinhaber: Henkel AG & Co. KGaA, 40589 Düsseldorf (DE)
(72) Erfinder: SCHÖPGENS, Jürgen, 41366 Schwalmtal (DE); MÜLLER, Burkhard, 40221 Düsseldorf (DE)
(86) Internationale Anmeldenummer: PCT/EP2015/063139
(87) Internationale Veröffentlichungsnummer: WO 2016/005143

(56) Entgegenhaltungen:
- EP-A1- 1 086 685
- DE-A1-102006 022 274
- DE-A1-102007 039 954

## Beschreibung

Die Erfindung betrifft Mehrkomponentenverpackungseinheiten (Kit-of-parts) zur reduktiven Entfärbung von gefärbten keratinischen Fasern, welche getrennt voneinander konfektioniert die Container (A), (B) und (C) enthalten. Hierbei enthält der Container (A) ein kosmetisches Mittel (a) mit mindestens einem ausgewählten Reduktionsmittel, der Container (B) enthält ein als Träger fungierendes kosmetisches Mittel (b), und der Container (C) enthält ein wässriges kosmetisches Mittel (c) mit mindestens einer anorganischen und/oder organischen Säure und mindestens einem zwitterionischen und/oder amphoteren Tensid. Ein weiterer Gegenstand der vorliegenden Erfindung ist ein Verfahren zur reduktiven Entfärbung von gefärbten Keratinfasern, bei welchem die zuvor beschriebene Mehrkomponenten-Verpackungseinheit verwendet wird.

Zubereitungen zum Tönen und Färben von Haaren sind ein wichtiger Typ von kosmetischen Mitteln. Sie können dazu dienen, die natürliche Haarfarbe gemäß den Wünschen der entsprechenden Person leicht oder stärker zu nuancieren, eine gänzlich andere Haarfarbe zu erzielen oder unerwünschte Farbtöne, wie beispielsweise Grautöne, zu überdecken. Übliche Haarfärbemittel werden, je nach gewünschter Farbe bzw. Dauerhaftigkeit der Färbung, entweder auf Basis von Oxidationsfarbstoffen oder auf Basis von direktziehenden Farbstoffen formuliert. Häufig werden auch Kombinationen von Oxidationsfarbstoffen und direktziehenden Farbstoffen zur Erzielung spezieller Nuancen eingesetzt.

Färbemittel auf Basis von Oxidationsfarbstoffen führen zu brillanten und dauerhaften Farbtönen. Sie bedingen allerdings den Einsatz starker Oxidationsmittel, wie beispielsweise Wasserstoffperoxid-Lösungen. Solche Färbemittel enthalten Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehrerer Kupplerkomponenten die eigentlichen Farbstoffe aus.

Färbemittel auf Basis von direktziehenden Farbstoffen werden häufig für temporäre Färbungen eingesetzt. Bei den direktziehenden Farbstoffen handelt es sich um Farbstoffmoleküle, die direkt auf das Haar aufziehen und keinen oxidativen Prozess zur Ausbildung der Farbe benötigen. Wichtige Vertreter dieser Farbstoffklasse sind beispielsweise Triphenylmethanfarbstoffe, Azofarbstoffe, Anthrachinonfarbstoffe oder Nitrobenzolfarbstoffe, welche jeweils kationische oder anionische Gruppen tragen können.

Bei all diesen Färbeprozessen kann es vorkommen, dass die Färbung aus verschiedenen Gründen ganz oder teilweise wieder rückgängig gemacht werden soll. Eine teilweise Entfernung der Färbung bietet sich beispielsweise an, wenn das Färbeergebnis auf den Fasern dunkler ausfällt als gewünscht. Andererseits kann auch eine vollständige Entfernung der Färbung in manchen Fällen gewünscht sein. So ist es beispielsweise denkbar, dass die Haare für einen konkreten Anlass in einer bestimmten Nuance gefärbt oder getönt werden sollen und nach einigen Tagen die ursprüngliche Farbe wieder zurückgewonnen werden soll.

Mittel und Verfahren zum Farbabzug sind bereits literaturbekannt. Ein aus dem Stand der Technik hinlänglich bekanntes Verfahren, Färbungen wieder rückgängig zu machen, ist die oxidative Entfärbung der gefärbten Haare, beispielsweise mithilfe eines üblichen Blondiermittels. Bei diesem Prozess können die Fasern aber durch den Einsatz starker Oxidationsmittel geschädigt werden. Ferner wurden auch reduktive Prozesse zum Farbabzug bereits beschrieben. So offenbart beispielsweise die europäische Patentanmeldung EP 1 300 136 A2 Verfahren zur Haarbehandlung, bei welchem die Haare in einem ersten Schritt gefärbt und in einem zweiten Schritt wieder reduktiv entfärbt werden. Hierbei erfolgt die reduktive Entfärbung durch Anwendung von einer Formulierung enthalten ein Dithionitsalz und ein Tensid. In der WO 2008/055756 A2 wird die reduktive Entfärbung von Keratinfasern mittels eines Gemisches aus einem Reduktionsmittel und einem Absorptionsmittel vorgenommen. Die Offenlegungsschrift DE 10 2007 039 954 A1 beschäftigt sich mit der Bereitstellung von reduktiven Entfärbemitteln, die Keratinfasern dauerhaft und ohne Nachdunklung entfärben sollen.

Bei Einsatz von reduktiven Entfärbemitteln findet die Entfärbung durch Reduktion der auf den Keratinfasern bzw. Haaren befindlichen Farbstoffe statt. Durch die Reduktion werden die Farbstoffe in der Regel in ihre reduzierten Leukoformen überführt. Bei diesem Vorgang werden die in den Farbstoffen vorhandenen Doppelbindungen reduziert, das chromophore System der Farbstoffe auf diese Weise unterbrochen und der Farbstoff in eine farblose Form überführt.

Ein generelles Problem bei den aus dem Stand der Technik bekannten reduktiven Entfärbemitteln besteht darin, dass die gefärbten Keratinfasern durch Einsatz des Reduktionsmittels zwar zunächst entfärbt werden können, der Farbabzug jedoch nicht von Dauer ist. Insbesondere bei oxidativ gefärbten Haaren, bei denen die Färbung durch Oxidationsfarbstoffvorprodukte vom Entwickler- und vom Kupplertyp auf dem Haar erzeugt wird, werden Färbungen mit teilweise sehr guten Echtheitseigenschaften erhalten. Bei Anwendung des reduktiven Entfärbemittels werden diese Farbstoffe nun reduktiv in ungefärbte Verbindungen überführt - die aufgrund ähnlich guter Echtheitseigenschaften jedoch nach wie vor auf dem Haar verbleiben.

Nach Abspülen des Reduktionsmittels und unter Einwirkung von Luftsauerstoff können diese reduzierten Formen nun nach und nach wieder zurückoxidiert werden. Bedingt durch diese Rückoxidation findet eine mehr oder weniger stark ausgeprägte Rückfärbung statt. Diese Rückfärbung entspricht in der Regel nicht dem Farbton, in dem die Keratinfasern zuvor gefärbt worden waren, sondern kann beliebig unattraktiv ausfallen und wird vom Anwender des Entfärbemittels daher umso weniger erwünscht.

Aufgabe der vorliegenden Erfindung war daher die Bereitstellung eines Entfärbemittels zur Entfärbung von gefärbten keratinischen Fasern, welches gefärbte Keratinfasern möglichst vollständig entfärbt. Die Entfärbung sollte lang anhaltend sein, und die entfärbten Keratinfasern sollten unter der Einwirkung von Luftsauerstoff keine Rückfärbung, keine Nuancenverschiebung und keine Nachdunklung erleiden. Das Entfärbemittel sollte insbesondere auf den Keratinfasern eine gute Entfärbeleistung zeigen, die zuvor mit oxidativen Färbemitteln auf Basis von Oxidationsfarbstoffvorprodukten vom Entwickler- und vom Kupplertyp gefärbt wurden.

Überraschenderweise hat sich nun herausgestellt, dass die nach Anwendung des reduktiven Entfärbemittels auftretende Rückdunklung wirkungsvoll unterdrückt werden kann, wenn die reduktiv entfärbten Keratinfasern nach der Entfärbung nochmals mit einem Nachbehandlungsmittel behandelt werden, welches ein oder mehrere Säuren aus der Gruppe anorganischen und/oder organischen Säuren und ein oder mehrere zwitterionische und/oder amphotere Tenside enthält. Ein erster Gegenstand der vorliegenden Erfindung ist eine Mehrkomponenten-Verpackungseinheit (Kit-of-parts) zur reduktiven Entfärbung von gefärbten keratinischen Fasern, welche getrennt voneinander konfektioniert
(I) einen Container (A) enthaltend ein kosmetisches Mittel (a),
(II) einen Container (B) enthaltend ein kosmetisches Mittel (b) und
(III) einen Container (C) enthaltend ein kosmetisches, wässriges Mittel (c) umfasst,
wobei
- das Mittel (a) in Container (A)
   (a1) ein oder mehrere Reduktionsmittel aus der Gruppe Natriumdithionit, Zinkdithionit, Kaliumdithionit, Natriumsulfit, Natriumhydrogensulfit, Kaliumsulfit, Kaliumhydrogensulfit, Ammoniumsulfit, Natriumthiosulfat, Kaliumthiosulfat, Ammoniumthiosulfat, Hydroxymethansulfinsäure, Aminomethansulfinsäure, Cystein, Thiomilchsäure, Sulfanylessigsäure (Thioglycolsäure) und/oder Ascorbinsäure enthält, und
- das Mittel (c) in Container (C)
   (c1) ein oder mehrere Säuren aus der Gruppe anorganischen und/oder organischen Säuren und
   (c2) ein oder mehrere zwitterionische und/oder amphotere Tenside enthält.

Die erfindungsgemäße Mehrkomponenten-Verpackungseinheit umfasst die getrennt voneinander konfektionierten Container (A) und (B), welche jeweils die Mittel (a) und (b) enthalten. Das Mittel (a) enthält mindestens ein Reduktionsmittel (a1). Bei dem Mittel (b) handelt es sich um eine Trägerformulierung, die wasserhaltig oder auch wasserfrei formuliert sein kann. Durch Vermischen der beiden Mittel (a) und (b) - d.h. durch Vermischen des reduktionsmittelhaltigen Mittels (a) mit dem Träger (b) - wird das anwendungsbereite Entfärbemittel hergestellt.

Weiterhin umfasst die erfindungsgemäße Mehrkomponenten-Verpackungseinheit einen dritten getrennt konfektionierten Container (C) mit einem Mittel (c). Bei dem Mittel (c) handelt es sich um ein Nachbehandlungsmittel, welches nach der Applikation des anwendungsbereiten Entfärbemittels auf die Keratinfasern aufgetragen werden soll. Durch Behandlung der Keratinfasern direkt nach der Entfärbung, d.h. direkt nach Applikation, Einwirken und Ausspülen des anwendungsbereiten Entfärbemittels, kann die Entfärbung effektiver gestaltet und insbesondere die zu einer Nachdunklung führende Rückoxidation wirkungsvoll verhindert werden.

Als insbesondere überraschend hat sich herausgestellt, dass die Entfärbewirkung bei Anwendung der erfindungsgemäßen Mehrkomponenten-Verpackungseinheit extrem lang anhaltend ist und dass auch die entfärbten Keratinfasern, die für Stunden bzw. Tage der Einwirkung von Luftsauerstoff ausgesetzt werden, keine Rückoxidation und keine Nachdunklung erleiden.

Unter keratinischen Fasern, keratinhaltigen Fasern oder Keratinfasern sind Pelze, Wolle, Federn und insbesondere menschliche Haare zu verstehen. Obwohl die erfindungsgemäßen Mittel in erster Linie zum Aufhellen und Färben von Keratinfasern bzw. menschlichen Haaren geeignet sind, steht prinzipiell einer Verwendung auch auf anderen Gebieten nichts entgegen.

Unter dem Begriff "gefärbte keratinische Fasern" werden Keratinfasern verstanden, die mit herkömmlichen, dem Fachmann bekannten kosmetischen Färbemitteln gefärbt wurden. Insbesondere sind unter den "gefärbten keratinischen Fasern" Fasern zu verstehen, die mit den aus dem Stand der Technik bekannten oxidativen Färbemitteln und/oder mit direktziehenden Farbstoffen gefärbt wurden. Es wird in diesem Zusammenhang ausdrücklich auf die bekannten Monographien, z. B. Kh. Schrader, Grundlagen und Rezepturen der Kosmetika, 2. Auflage, Hüthig Buch Verlag, Heidelberg, 1989, verwiesen, die das entsprechende Wissen des Fachmannes wiedergeben.

Die Mittel enthalten die erfindungswesentlichen Inhaltsstoffe bevorzugt in einem kosmetischen Träger (Mittel (a) und (b)) bzw. in einem wässrigen kosmetischen Träger (Mittel (c)). Hierbei kann es sich beispielsweise um einen geeigneten wässrigen oder wässrig-alkoholischen Träger handeln. Zum Zwecke der reduktiven Entfärbung können solche Träger beispielsweise Cremes, Emulsionen, Gele oder auch tensidhaltige schäumende Lösungen, wie beispielsweise Shampoos, Schaumaerosole, Schaumformulierungen oder andere Zubereitungen, die für die Anwendung auf dem Haar geeignet sind, sein. Besonders bevorzugt handelt es sich bei den Mitteln zum reduktiven Farbabzug von keratinischen Fasern um Cremes, Emulsionen oder fließfähige Gele. Insbesondere das Mittel (a) kann auch wasserfrei konfektioniert werden und beispielsweise in fester Form, als Pulver oder Paste vorliegen. Das Mittel (a) kann weiterhin auch einen lösungsmittelhaltigen Träger oder einen Träger aus Fettbestandteilen wie beispielsweise Fettalkohole, Fettsäureester, Kohlenwasserstoffe, Silikonöle und/oder hydrophobe Öle umfassen.

### Mittel (a) in Container (A)

Die erfindungsgemäße Mehrkomponenten-Verpackungseinheit (Kit-of-parts) umfasst einen ersten getrennt konfektionierten Container (A) mit einem kosmetischen Mittel (a). Das Mittel (a) ist dadurch gekennzeichnet, dass es als ersten erfindungswesentlichen Inhaltsstoff (a1) mindestens ein Reduktionsmittel aus der Gruppe Natriumdithionit, Zinkdithionit, Kaliumdithionit, Natriumsulfit, Natriumhydrogensulfit, Kaliumsulfit, Kaliumhydrogensulfit, Ammoniumsulfit, Natriumthiosulfat, Kaliumthiosulfat, Ammoniumthiosulfat, Hydroxymethansulfinsäure, Aminomethansulfinsäure, Cystein, Thiomilchsäure, Sulfanylessigsäure (Thioglycolsäure) und/oder Ascorbinsäure enthält. Bei Natriumdithionit handelt es sich um ein anorganischen Reduktionsmittel mit der Summenformel Na₂S₂O₄ und der CAS-Nr. 7775-14-6.

Bei Zinkdithionit handelt es sich um ein anorganisches Reduktionsmittel mit der Summenformel ZnS₂O₄ und der CAS-Nr. 7779-86-4.

Bei Kaliumdithionit handelt es sich um ein anorganisches Reduktionsmittel mit der Summenformel K₂S₂O₄ und der CAS-Nr. 14293-73-3.

Bei Natriumsulfit handelt es sich um ein anorganisches Reduktionsmittel mit der Summenformel Na₂SO₃ und der CAS-Nr. 7757-83-7.

Bei Natriumhydrogensulfit handelt es sich um ein anorganisches Reduktionsmittel mit der Summenformel NaHSO₃ und der CAS-Nr. 7631-90-5. Natriumhydrogensulfit wird bevorzugt in Form einer wässrigen Lösung eingesetzt.

Bei Kaliumsulfit handelt es sich um ein ein anorganisches Reduktionsmittel mit der Summenformel K₂SO₃ und der CAS-Nr. 10117-38-1.

Bei Kaliumhydrogensulfit handelt es sich um ein anorganisches Reduktionsmittel mit der Summenformel KHSO₃ und der CAS-Nr. 7773-03-7. Kaliumhydrogensulfit wird bevorzugt in Form einer wässrigen Lösung eingesetzt.

Bei Ammoniumsulfit handetl es sich um ein anorganisches Reduktionsmittel mit der Summenformel (NH₄)₂SO₃ und der CAS-Nr. 10196-04-0.

Bei Natriumthiosulfat handelt es sich um ein anorganisches Reduktionsmittel mit der Summenformel

Na₂S₂O₃ und der CAS-Nr. 7772-98-7.

Bei Kaliumthiosulfat handelt es sich um ein anorganisches Reduktionsmittel mit der Summenformel K₂S₂O₃ und der CAS-Nr. 10294-66-3.

Bei Ammoniumthiosulfat handelt es sich um ein anorganisches Reduktionsmittel mit der Summenformel (NH₄)₂S₂O₃ und der CAS-Nr. 7783-18-8.

Bei Hydroxymethansulfinsäure handelt es sich um ein organisches Reduktionsmittel mit der Formel HO-CH₂-S(O)OH und der CAS-Nr. 79-25-4. Alternativ wird Hydroxymethansulfinsäure auch als Formaldehydsulfoxylsäure bezeichnet. Erfindungsgemäß ist sowohl der Einsatz der Hydroxymethansulfinsäure selbst als auch der Einsatz der physiologisch verträglichen Salze von Hydroxymethansulfinsäure, beispielsweise des Natriumsalzes und/oder des Zinksalzes. Der Einsatz von Natriumformaldehydsulfoxylat (Natrium Hydroxymethansulfinat, dem Natriumsalz der Hydroxymethansulfinsäure) und/oder Zinkformeladehydsulfoxylat (Zink Hydroxymethansulfinat, dem Zinksalz der Hydroxymethansulfinsäure) ist demnach ebenfalls erfindungsgemäß.

Bei Aminomethansulfinsäure handelt es sich um ein organisches Reduktionsmittel mit der Formel H₂N-CH₂-S(O)OH und der CAS-Nr. 118201-33-5. Erfindungsgemäß ist sowohl der Einsatz der Aminomethansulfinsäure selbst als auch der Einsatz der physiologisch verträglichen Salze von Aminomethansulfinsäure, beispielsweise des Natriumsalzes und/oder des Zinksalzes. Der Einsatz von Natrium Aminomethansulfinat (dem Natriumsalz der Aminomethansulfinsäure) und/oder Zink Aminomethansulfinat (dem Zinksalz der Aminomethansulfinsäure) ist deshalb ebenfalls erfindungsgemäß.

Unter Cystein (2-Amino-3-sulfanylpropionsäure) wird erfindungsgemäß D-Cystein, L-Cystein und/oder ein Gemisch aus D- und L-Cystein verstanden.

Unter Thiomilchsäure (2-Sulfanylpropionsäure) wird D-Thio-Milchsäure, L-Thio-Milchsäure und/oder ein Gemisch aus D- und L-Thiomilchsäure verstanden. Erfindungsgemäß ist sowohl der Einsatz der Thiomilchsäure selbst als auch der Einsatz von Thiomilchsäure in Form eines physiologisch verträglichen Salzes hiervon. Ein bevorzugtes Salz der Thiomilchsäure ist Ammoniumthiolactat.

Bei Ammoniumthiolactat handelt es sich um das Ammoniumsalz der Thiomilchsäure (d.h. das Ammoniumsalz der 2-Sulfanylpropionsäure) (Formel XX).

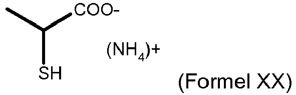

Von der Definition Ammoniumthiolactat mit umfasst sind sowohl die Ammoniumsalze der D-Thiomilchsäure als auch die Ammoniumsalze der L-Thiomilchsäure sowie deren Gemische. Unter Sulfanylessigsäure (Thioglycolsäure, 2-Mercapto-essigäure) wird ein organisches Reduktionsmittel der Formel HS-CH₂-COOH verstanden, die Verbindung besitzt die CAS-Nr. 68-11-1. Auch bei Thioglycolsäure ist sowohl der Einsatz von Thioglycolsäure selbst auch der Einsatz eines physiologisch verträglichen Salzes der Thioglycolsäure erfindungsgemäß. Als physiologisch verträgliche Salze der Thiolgycolsäure können beispielsweis Natriumthioglycolat, Kaliumthioglycolat und/oder Ammoniumthioglycolat eingsetzt werden. Ammoniumthioglylat ist ein bevorzugtes physiologisch vertägliches Salze der Thioglycolsäure.

Bei Ammoniumthioglycolat handelt es sich um das Ammoniumsalz der Thiglycolsäure (d.h. das Ammoniumsalz der Sulfanylessigsäure) (Formel XXX).

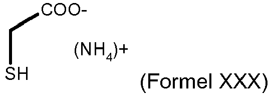

Unter Ascorbinsäure wird erfindungsgemäß insbesondere (*R*)-5-[(*S*)-1,2-Dihydroxyethyl]-3,4-dihydroxy-5*H*-furan-2-on (weitere Alternativnamen: Vitamin C, L-Ascorbinsäure) mit der CAS-Nr. 50-81-7 verstanden.

Als besonders gut geeignet zur reduktiven Entfärbung von oxidativ gefärbten Haaren haben sich die Reduktionsmittel Natriumdithionit, Zinkdithionit, Kaliumdithionit, Natriumsulfit, Natriumhydrogensulfit, Kaliumsulfit, Kaliumhydrogensulfit, Ammoniumsulfit, Natriumthiosulfat, Kaliumthiosulfat und/oder Ammoniumthiosulfat herausgestellt. Werden diese vorgenannten bevorzugten Reduktionsmittel (a1) mit einem Mittel (c) nachbehandelt, welche die erfindungsgemäße Kombination aus Säuren (c1) und den amphoteren bzw. zwitterionischen Tensiden (c2) enthält, so konnte eine besonders effektive Entfärbung und eine besonders wirksame Verhinderung der Rückoxidation bei den entfärbten Haarsträhnen beobachtet werden. Auf diese Weise konnte ein Nachdunkeln der entfärbten Keratinfasern über einen besonders langen Zeitraum verhindert werden.

Weiterhin werden das bzw. die Reduktionsmittel aus der Gruppe (a) bevorzugt in bestimmten Mengenbereichen eingesetzt. Um eine optimale Entfärbewirkung zu erhalten, ist es bevozugt, wenn das Entfärbemittel das oder die Reduktionsmittel (a1) in einer Gesamtmenge von 25,0 bis 100 Gew.-%, bevorzugt von 45,0 bis 100 Gew.-%, weiter bevorzugt von 65,0 bis 100 Gew.-% und besonders bevorzugt von 85,0 bis 100 Gew.-% - bezogen auf das Gesamtgewicht des Mittels (a) - enthält.

Eine besonders bevorzugte Mehrkomponenten-Verpackungseinheit (Kit-of-parts) ist weiterhin dadurch dadurch gekennzeichnet dass
- das Mittel (a) in Container (A)
   (a1) ein oder mehrere Reduktionsmittel aus der Gruppe Natriumdithionit, Zinkdithionit, Kaliumdithionit, Natriumsulfit, Natriumhydrogensulfit, Kaliumsulfit, Kaliumhydrogensulfit, Ammoniumsulfit, Natriumthiosulfat, Kaliumthiosulfat und/oder Ammoniumthiosulfat in einer Gesamtmenge von 25,0 bis 100 Gew.-%, bevorzugt von 45,0 bis 100 Gew.-%, weiter bevorzugt von 65,0 bis 100 Gew.-% und besonders bevorzugt von 85,0 bis 100 Gew.-% - bezogen auf das Gesamtgewicht des Mittels (a) - enthält.
      Besonders bevorzugt ist weiterhin auch eine Mehrkomponenten-Verpackungseinheit (Kit-of-parts) zur reduktiven Entfärbung von gefärbten keratinischen Fasern, die dadurch gekennzeichnet ist, dass
- das Mittel (a) in Container (A)
   (a1) ein oder mehrere Reduktionsmittel aus der Gruppe Natriumdithionit, Zinkdithionit, Kaliumdithionit, Natriumthiosulfat, Kaliumthiosulfat und/oder Ammoniumthiosulfat in einer Gesamtmenge von 25,0 bis 100 Gew.-%, bevorzugt von 45,0 bis 100 Gew.-%, weiter bevorzugt von 65,0 bis 100 Gew.-% und besonders bevorzugt von 85,0 bis 100 Gew.-% - bezogen auf das Gesamtgewicht des Mittels (a) - enthält.
      Weiterhin hat es sich als besonders vorteilhaft herausgestellt, wenn die erfindungsgemäßen Mittel bestimmte Kombinationen aus Reduktionsmitteln aus der Gruppe (a1) enthalten, da bei bestimmten Kombinationen ein ganz besonders starker entfärbender Effekt auftritt. Besonders vorteilhaft ist in diesem Zusammenhang der Einsatz von zwei verschiedenen Reduktionsmitteln aus der Gruppe (a1), wobei das Entfärbemittel
   (a11) ein erstes Reduktionsmittel enthält, das aus der Gruppe Natriumdithionit, Zinkdithionit, Kaliumdithionit, Natriumthiosulfat, Kaliumthiosulfat und/oder Ammoniumthiosulfat ausgewählt wird, und zusätzlich
   (a12) ein zweites Reduktionsmittel enthält, das aus der Gruppe Natriumsulfit, Natriumhydrogensulfit, Kaliumsulfit, Kaliumhydrogensulfit und/oder Ammoniumsulfit ausgewählt wird.

Mit anderen Worten besonders bevorzugt ist im Rahmen dieser Ausführungsform eine Mehrkomponenten-Verpackungseinheit zur reduktiven Entfärbung von gefärbten keratinischen Fasern, insbesondere menschlichen Haaren, wobei
- das Mittel (a) in Container (A)
   (a11) ein erstes Reduktionsmittel, das aus der Gruppe Natriumdithionit, Zinkdithionit, Kaliumdithionit, Natriumthiosulfat, Kaliumthiosulfat und/oder Ammoniumthiosulfat ausgewählt wird, und zusätzlich
   (a12) ein zweites Reduktionsmittel, das aus der Gruppe Natriumsulfit, Natriumhydrogensulfit, Kaliumsulfit, Kaliumhydrogensulfit und/oder Ammoniumsulfit ausgewählt wird, enthält.

### Mittel (b) in Container (B)

Die erfindungsgemäße Mehrkomponenten-Verpackungseinheit umfasst einen zweiten getrennt konfektionierten Container (B), welcher ein Mittel (b) enthält. Bei diesem Mittel (b) handelt es sich um eine kosmetische Trägerformulierung, welche bevorzugt wässrig oder wässrig-alkoholisch sein kann. Ganz besonders bevorzugt ist das Mittel (b) wässrig.

Eine ganz besonders bevorzugte Mehrkomponenten-Verpackungseinheit (Kit-of-parts) ist weiterhin dadurch dadurch gekennzeichnet dass das Mittel (b) in Container (B)
(b1) Wasser enthält.

Kurz vor dem Entfärbevorgang werden die Mittel (a) und (b) zur Herstellung des anwendungsbereiten Entärbemittels vermischt.

Das Mittel (b) wird bevorzugt als flüssige Zubereitung bereitgestellt, dem weitere oberflächenaktive Substanzen zugesetzt werden können. Sie sind bevorzugt aus anionischen, zwitterionischen, amphoteren und nichtionischen Tensiden und Emulgatoren ausgewählt.

Als anionische Tenside kann das Mittel (b) beispielsweise Fettsäuren, Alkylsulfate, Alkylethersulfate und Ethercarbonsäuren mit 10 bis 20 C-Atomen in der Alkylgruppe und bis zu 16 Glykolethergruppen im Molekül enthalten.

Das Mittel (b) kann auch ein oder mehrere zwitterionische Tenside wie beispielsweise Betaine, N-Alkyl-N,N-dimethylammonium-glycinate, N-Acyl-aminopropyl-N,N-dimethylammoniumglycinate, und 2-Alkyl-3-carboxymethyl-3-hydroxyethyl-imidazoline enthalten.

Erfindungsgemäß geeignete Mittel (b) sind weiterhin dadurch gekennzeichnet, dass das Mittel (b) zusätzlich mindestens ein amphoteres Tensid enthält. Bevorzugte amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside sind N-Kokosalkylaminopropionat, as Kokosacylaminoethylaminopropionat und C₁₂-C₁₈-Acylsarcosin.

Weiterhin hat es sich als vorteilhaft erwiesen, wenn das Mittel (b) weitere, nichtionogene grenzflächenaktive Stoffe, enthält. Bevorzugte nichtionische Tenside sind Alkylpolyglykoside sowie Alkylenoxid-Anlagerungsprodukte an Fettalkohole, Fettsäuren und Fettsäureglyceride mit jeweils 2 bis 50 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten. Ganz besonders bevorzugt ist es, wenn das Mittel (b) als nichtionisches Tensid ein ethoxyliertes Rizinusöl mit jeweils 2 bis 50 Mol Ethylenoxyid pro Mol Fettsäure oder ein ethoxyliertes, hydriertes Rizinusöl mit jeweils 2 bis 50 Mol Ethylenoxyid pro Mol Fettsäure enthält. Der Einsatz von PEG-40 Castor Oil ist in diesem Zusammenhang besonders bevorzugt.

Die nichtionischen, zwitterionischen oder amphoteren Tenside werden in Anteilen von 0,1 bis 15,0 Gew.%, bevorzugt 0,5 bis 10,0 Gew.% und ganz besonders bevorzugt von 0,7 bis 5,0 Gew.%, bezogen auf die Gesamtmenge des Mittels, eingesetzt.

Zur Optimierung der Entfärbewirkung besitzt das anwendungsbereite Entfärbemittel - d.h. die Mischung der Mittel (a) und (b) - bevorzugt einen sauren pH-Wert. Daher wird auch das Mittel (b) bevorzugt auf einen sauren pH-Wert von 1 bis 6, bevorzugt von 1,3 bis 4,5, weiter bevorzugt von 1,6 bis 4,0 und besonders bevorzugt von 2,0 bis 3,6 besitzt, eingestellt. Zur Einstellung des sauren pH-Wertes enthält das Mittel (b) bevorzugt ein oder mehrere organische und/oder anorganische Säuren. Eine ganz besonders bevorzugte Mehrkomponenten-Verpackungseinheit (Kit-of-parts) ist weiterhin dadurch dadurch gekennzeichnet, dass das Mittel (b) in Container (B)
(b1) Wasser und
(b2) ein oder mehrere Säuren aus der Gruppe anorganischen und/oder organischen Säuren enthält.

Als insbesondere geeignet haben sich eine oder mehrere Säuren aus der Gruppe Zitronensäure, Weinsäure, Äpfelsäure, Milchsäure, Essigsäure, Schwefelsäure, Salzsäure, Phosphorsäure, Methansulfonsäure, Benzoesäure Malonsäure, Oxalsäure und/oder 1-Hydroxyethan-1,1-diphosphonsäure erwiesen. Malonsäure, Oxalsäure und/oder 1-Hydroxyethan-1,1-diphosphonsäure werden mit ganz besonderem Vorzug eingesetzt.

Ebenfalls besonders bevorzugt ist daher auch eine Mehrkomponenten-Verpackungseinheit (Kit-of-parts), welche dadurch gekennzeichnet ist, dass das Mittel (b) in Container (B)
(b1) Wasser und
(b2) ein oder mehrere Säuren aus der Gruppe Zitronensäure, Weinsäure, Äpfelsäure, Milchsäure Methansulfonsäure, Malonsäure, Oxalsäure und/oder 1-Hydroxyethan-1,1-diphosponsäure enthält.

### Mittel (c) in Container (C)

Die erfindungsgemäße Mehrkomponenten-Verpackungseinheit umfasst weiterhin einen dritten Container (C), welcher getrennt konfektioniert das Mittel (c) enthält. Bei dem Mittel (c) handelt es sich um ein Nachbehandlungsmittel, welches nach Applikation, Einwirken und vorzugsweise nach Abspülen des anwendungsbereiten Entfärbemittels (d.h. des Gemisches der Mittel (a) und (b)) auf die Keratinfasern aufgetragen werden soll.

Durch die Anwendung des Nachbehandlungsmittels (c) wird die Rückoxidation bzw. Nachdunklung verhindert, die ansonsten üblicherweise immer dann auftritt, wenn die reduktiv behandelten, entfärbten und durch Auswaschen vom Reduktionsmittel befreiten Keratinfasern der Einwirkung von Luftsauerstoff ausgesetzt werden. Um die Nachdunklung in optimaler Weise zu verhindern, wird das Nachbehandlungsmittel bevorzugt direkt nach dem Ausspülen des Entfärbemittels (d.h. des Gemisches der Mittel (a) und (b)) aufgetragen.

Erfindungswesentlich für das Mittel (c) ist dessen Gehalt an mindestens einer Säure aus der Gruppe der anorganischen und/oder organischen Säuren (c1) und mindestens einem zwitterionischen und/oder amphoteren Tensid (c2).

Die Fähigkeit, die Rückoxidation zu unterdrücken, hängt hierbei wesentlich von der Wahl der Säure (c1) und des amphoteren bzw. zwitterionischen Tensids (c2) ab. Beide Komponenten (c1) und (c2) wirken bei der Verhinderung der Nachdunklung zusammen, wobei sich spezielle Säuren und ausgewählte Tenside als besonders effektiv erwiesen haben.

Geeignete Säuren werden in diesem Zusammenhang aus der Gruppe ausgewählt, die aus Zitronensäure, Weinsäure, Äpfelsäure, Milchsäure, Essigsäure, Hydroxyethan-1,1-diphosphonsäure, Methansulfonsäure, Benzosäure, Salzsäure, Schwefelsäure, Phosphorsäure, Malonsäure und/oder Oxalsäure gebildet wird.

Die Säure bzw. die Säuren werden hierbei sowohl zur Einstellung des für das Nachbehandlungsmittel (c) optimalen pH-Wertes als auch zur Unterdrückung der Nachdunklung eingesetzt. Hierbei hat sich der Einsatz bestimmter Mengenbereiche als besonders gut zur Lösung der erfindungsgemäßen Aufgabenstellung erwiesen.

Bevorzugt werden die Säure bzw. die Säuren in einer Gesamtmenge von von 2,0 bis 20,0 Gew.-%, bevorzugt von 3,0 bis 18,0 Gew.-%, weiter bevorzugt von 3,5 bis 16,0 Gew.-% und besonders bevorzugt von 4,0 bis 14,0 Gew.-% - bezogen auf das Gesamtgewicht des Mittels (c) - eingesetzt.

Eine bevorzugte Mehrkomponenten-Verpackungseinheit (Kit-of-parts) ist weiterhin dadurch gekennzeichnet, dass das Mittel (c) in Container (C)
(c1) ein oder mehrere Säuren aus der Gruppe Zitronensäure, Weinsäure, Äpfelsäure, Milchsäure, Essigsäure, Hydroxyethan-1,1-diphosphonsäure, Methansulfonsäure, Benzosäure, Salzsäure, Schwefelsäure, Phosphorsäure, Malonsäure und/oder Oxalsäure in einer Gesamtmenge von 2,0 bis 20,0 Gew.-%, bevorzugt von 3,0 bis 18,0 Gew.-%, weiter bevorzugt von 3,5 bis 16,0 Gew.-% und besonders bevorzugt von 4,0 bis 14,0 Gew.-% - bezogen auf das Gesamtgewicht des Mittels (c) - enthält.

Als ganz besonders effektiv bei der Verhinderung der Rückoxidation erwiesen haben sich die Säuren, die aus der Gruppe Methansulfonsäure, Malonsäure und/oder Oxalsäure ausgewählt werden. Daher sind die Säuren Methansulfonsäure, Malonsäure und Oxalsäure nochmals ganz besonders bevorzugt.

Eine ganz besonders bevorzugte Mehrkomponenten-Verpackungseinheit (Kit-of-parts) ist weiterhin dadurch dadurch gekennzeichnet, dass das Mittel (c) in Container (C)
(c1) ein oder mehrere Säuren aus der Gruppe Methansulfonsäure, Malonsäure und/oder Oxalsäure in einer Gesamtmenge von 2,0 bis 20,0 Gew.-%, bevorzugt von 3,0 bis 18,0 Gew.-%, weiter bevorzugt von 3,5 bis 16,0 Gew.-% und besonders bevorzugt von 4,0 bis 14,0 Gew.-% - bezogen auf das Gesamtgewicht des Mittels (c) - enthält.

Im Zuge der zu dieser Erfindung führenden Arbeiten hat sich überraschenderweise herausgestellt, dass das Entfärbe-Ergebnis und dessen Wirkdauer noch weiter verbessert werden können, wenn das Entfärbemittel (c) zwei verschiedene Säuren enthält.

Eine ganz besonders bevorzugte Mehrkomponenten-Verpackungseinheit (Kit-of-parts) ist weiterhin dadurch dadurch gekennzeichnet, dass das Mittel (c) in Container (C)
(c1) als Säuren Oxalsäure und/oder Malonsäure sowie eine weitere Säure aus der Gruppe Zitronensäure, Weinsäure, Äpfelsäure, Milchsäure, Essigsäure, Hydroxyethan-1,1-diphosphonsäure, Methansulfonsäure, Benzosäure, Salzsäure, Schwefelsäure, Phosphorsäure enthält.

Die allerbesten Entfärbeergebnisse wurden mit den Säuregemischen

Oxalsäure/Methansulfonsäure und Malonsäure/Methansulfonsäure erhalten.

Eine explizit ganz besonders bevorzugte Mehrkomponenten-Verpackungseinheit (Kit-of-parts) ist weiterhin dadurch dadurch gekennzeichnet, dass das Mittel (c) in Container (C)
(c1) als Säuren Oxalsäure und/oder Malonsäure sowie als weitere Säure Methansulfonsäure enthält.

Als zweiten erfindungswesentlichen Bestandteil enthält das Mittel (c) in Container (C) ein oder mehrere zwitterionische und/oder amphotere Tenside (c2).

Unter Tensiden werden amphiphile (bifunktionelle) Verbindungen mit mindestens einem hydrophoben Rest und mindestens einem hydrophilen Molekülteil verstanden. Bei dem hydrophoben Molekülteil handelt es sich zumeist um eine Kohlenwasserstoffkette mit ca. 10 bis 30 Kohlenstoff-Atomen.

Im Fall der zwitterionischen Tenside umfasst der hydrophile Molekülteil eine zwitterionische Struktureinheit, d.h. eine Struktureinheit, die sowohl einen kationisch geladenen als auch einen anionisch geladenen Molekülteil umfasst. Erfindungsgemäße zwitterionische Tenside (c2) sind dadurch gekennzeichnet, dass sie einen kationisch geladenen Molekülteil in Form einer quartären Ammoniumgruppe besitzen und ihr anionischer Molekülteil in Form einer Gruppierung -COO- oder -SO₃⁻ vorliegt.

Eine Ammoniumgruppe ist quartär, wenn eine Gruppierung des Typs (R₁R₂R₃R₄N)⁺ vorliegt, d.h. wenn alle vier H-Atome des NH₄-Ions, von dem die quartäre Ammoniumgruppe abgeleitet ist, durch organische Reste R ersetzt wird.

Die Gruppierung -SO3⁻ des zwitterionischen Tensids kann direkt an ein Kohlenstoffatom gebunden vorliegen. In diesem Fall handelt es sich bei dem anionischen Teil der zwitterionischen Verbindung um eine deprotonierte Sulfonsäuregruppierung der Formel R₅R₆R₇C-SO3⁻, wobei die Reste R5, R6, R7 den restlichen Teil des erfindungsgemäßen zwitterionischen Tensids darstellen.

Es ist jedoch ebenfalls auch erfindungsgemäß, wenn die Gruppierung -SO₃⁻ über ein Sauerstoffatom an ein Kohlenstoffatom gebunden vorliegt, in diesem Fall stellt der anionische Teil des zwitterionischen Tensids einen deprotonierten Schwefelsäureester der Formel R₅R₆R₇C-O-SO₃⁻ dar, wobei die Reste R5, R6, R7 wieder den restlichen Teil des erfindungsgemäßen zwitterionischen Tensids darstellen.

Erfindungsgemäß bevorzugt ist es, wenn die Gruppierung -SO3⁻ direkt an ein Kohlenstoffatom gebunden, d.h. in Form einer deprotonierten Sulfonsäuregruppierung, vorliegt.

Unter amphoteren Tensiden werden solche oberflächenaktiven Verbindungen verstanden, die außer einer C10-C30-Alkyl- oder Acylgruppe im Molekül mindestens eine freie Aminogruppe und mindestens eine -COOH- oder -SO3H-Gruppe enthalten und die zur Ausbildung innerer Salze befähigt sind. Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 10 bis 30 C-Atomen in der Alkylgruppe. Besonders bevorzugte amphotere Tenside sind das N-Kokosalkylaminopropionat, das Kokosacylaminoethylaminopropionat und das C12-18-Acylsarcosin.

Ganz besonders bevorzugt enthält das Mittel (c) ein oder mehrere zwitterionische Tenside (c2). Aus diesem Grund ist auch die Mehrkomponenten-Verpackungseinheit (Kit-of-parts) zur reduktiven Entfärbung von gefärbten keratinischen Fasern ganz besonders geeignet, welche getrennt voneinander konfektioniert
(I) einen Container (A) enthaltend ein kosmetisches Mittel (a)
(II) einen Container (B) enthaltend ein kosmetisches Mittel (b) und
(III) einen Container (C) enthaltend ein kosmetisches, wässriges Mittel (c) umfasst,
wobei
- das Mittel (a) in Container (A)
   (a1) ein oder mehrere Reduktionsmittel aus der Gruppe Natriumdithionit, Zinkdithionit, Kaliumdithionit, Natriumsulfit, Natriumhydrogensulfit, Kaliumsulfit, Kaliumhydrogensulfit, Ammoniumsulfit, Natriumthiosulfat, Kaliumthiosulfat, Ammoniumthiosulfat, Hydroxymethansulfinsäure, Aminomethansulfinsäure, Cystein, Thiomilchsäure, Sulfanylessigsäure (Thioglycolsäure) und/oder Ascorbinsäure enthält,
- das Mittel (b) in Container (B)
   (b1) Wasser und
   (b2) ein oder mehrere Säuren aus der Gruppe anorganischen und/oder organischen Säuren enthält, und
- das Mittel (c) in Container (C)
   (c1) ein oder mehrere Säuren aus der Gruppe anorganischen und/oder organischen Säuren und
   (c2) ein oder mehrere zwitterionische Tenside enthält.

Über einen besonders langen Zeitraum ließ sich eine Nachdunklung der entfärbten Keratinfasern verhindern, wenn als erfindungsgmäßes zwitterionisches Tensid (c2) ein oder mehrere zwitterionische Tenside der Formeln (I) bis (IV) eingesetzt wurden 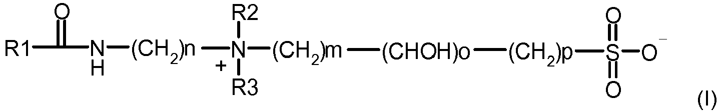 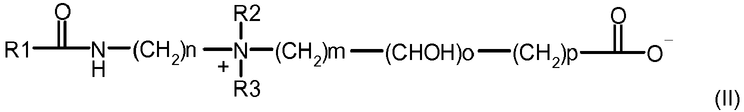 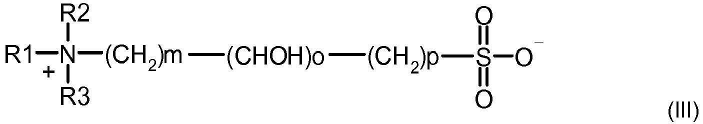 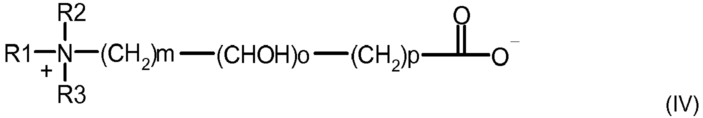 worin
- R1: jeweils unabhängig voneinander für eine lineare oder verzweigte C₉-C₂₉-Alkylgruppe, eine lineare oder verzweigte C₉-C₂₉-Alkenylgruppe oder eine lineare oder verzweigte Hydroxy-C₉-C₂₉-Alkylgruppe steht,
- R2, R3: jeweils unabhängig voneinander für eine C₁-C₆-Alkylgruppe, eine C₂-C₆-Alkenylgruppe oder eine Hydroxy-C₂-C₆-alkylgruppe stehen,
- n: jeweils unabhängig voneinander für eine ganze Zahl von 1 bis 6 steht,
- m: jeweils unabhängig voneinander für eine ganze Zahl von 0 bis 6 steht,
- o: jeweils unabhängig voneinander für eine ganze Zahl von 0 bis 6 steht,
- p: jeweils unabhängig voneinander für eine ganze Zahl von 0 bis 6 steht,
mit der Maßgabe, das die Summe aus m, o und p jeweils mindestens 1 beträgt.

Eine bevorzugte Mehrkomponenten-Verpackungseinheit (Kit-of-parts) ist weiterhin dadurch dadurch gekennzeichnet, dass das Mittel (c) in Container (C)
(c2) ein oder mehrere zwitterionische Tenside ausgewählt aus der Gruppe der Formeln (I) bis (IV) enthält, 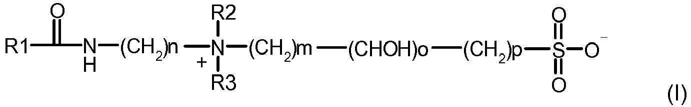 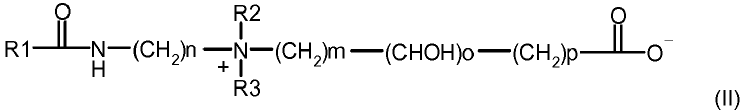 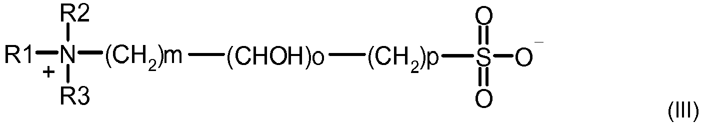 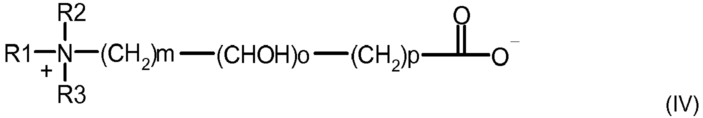 worin
   - R1: jeweils unabhängig voneinander für eine lineare oder verzweigte C₉-C₂₉-Alkylgruppe, eine lineare oder verzweigte C₉-C₂₉-Alkenylgruppe oder eine lineare oder verzweigte Hydroxy-C₉-C₂₉-Alkylgruppe steht,
   - R2, R3: jeweils unabhängig voneinander für eine C₁-C₆-Alkylgruppe, eine C₂-C₆-Alkenylgruppe oder eine Hydroxy-C₂-C₆-alkylgruppe stehen,
   - n: jeweils unabhängig voneinander für eine ganze Zahl von 1 bis 6 steht,
   - m: jeweils unabhängig voneinander für eine ganze Zahl von 0 bis 6 steht,
   - o: jeweils unabhängig voneinander für eine ganze Zahl von 0 bis 6 steht,
   - p: jeweils unabhängig voneinander für eine ganze Zahl von 0 bis 6 steht,
   mit der Maßgabe, das die Summe aus m, o und p jeweils mindestens 1 beträgt.

Innerhalb der Gruppe der zwitterionischen Tenside der Formeln (I) bis (IV) sind die Tenside der Formeln (I) und/oder (II) ganz besonders bevorzugt.

Insbesondere bevorzugt ist der Einsatz von zwitterionischen Tensiden der Formel (I), bei welchen der Rest R1 für eine lineare C₁₁-Alkylgruppe, eine lineare C₁₃-Alkylgruppe, eine lineare C₁₅-Alkylgruppe, eine lineare C₁₇-Alkylgruppe oder eine lineare C₁₉-Alkylgruppe, eine lineare, einfach ungesättigte C₁₁-Alkenylgruppe, eine lineare, einfach ungesättigte C₁₃-Alkenylgruppe, eine lineare, einfach ungesättigte C₁₅-Alkylgruppe, eine lineare, einfach ungesättigte C₁₇-Alkenylgruppe oder eine lineare, einfach ungesättigte C₁₉-Alkenylgruppe steht.

Weiterhin ist es ganz besonders bevorzugt, wenn n für die Zahl 3 steht.

Die Reste R2 und R3 stehen bevorzugt unabhängig voneinander für eine C₁-C₆-Alkylgruppe, besonders bevorzugt stehen R2 und R3 beide für eine Methylgruppe.
m steht ganz besonders bevorzugt für die Zahlen 1 oder 2.
o steht ganz besonders bevorzugt für die Zahl 1.
p steht ganz besonders bevorzugt für die Zahlen 1 oder 2.

Eine ganz besonders bevorzugte Mehrkomponenten-Verpackungseinheit (Kit-of-parts) ist weiterhin dadurch dadurch gekennzeichnet, dass das Mittel (c) in Container (C)
(c2) ein oder mehrere zwitterionische Tenside der Formel (I) enthält, 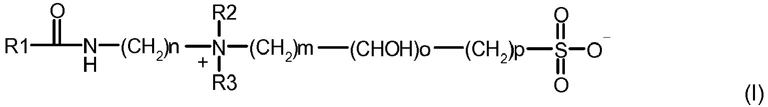 worin
   - R1: für eine lineare oder verzweigte C₁₀-C₃₀-Alkylgruppe oder eine lineare oder verzweigte, ein- oder mehrfach ungesättigte C₁₀-C₃₀-Alkenylgruppe steht,
   - R2, R3: jeweils unabhängig voneinander für eine C₁-C₆-Alkylgruppe stehen,
   - n: für eine ganze Zahl von 1 bis 6, bevorzugt für die Zahl 3, steht,
   - m: für eine ganze Zahl von 1 bis 6 steht,
   - o: für eine ganze Zahl von 1 bis 6 steht, und
   - p: für eine ganze Zahl von 1 bis 6 steht.

Die zwitterionischen Tenside dieses ganz besonders bevorzugten Typs der Formel (I) sind auch unter dem Namen "Amidopropylhydroxysultaine" bekannt:
Laurylamidpropylhydroxysultain ist ein zwitterionischesTensid der Formel (I), wobei R1 für C11-Alkyl, R2 für Methyl, R3 für Methyl, n für 3, m für 1, o für 1 und p für 1 steht.

Cocamidopropylhydroxysultain stellt ein Gemisch von Verbindungen der Formel (I) dar, bei denen R1 steht für C11-Alkyl bis C17-Alkyl, R2 für Methyl, R3 für Methyl, n für 3, m für 1, o für 1 und p für 1.

Oleamidopropylhydroxysultain ist ein zwitterionisches Tensid der Formel (I), wobei R1 für eine einfach ungesättigte C17-Alkenylgruppe steht (die Doppelbindung liegt zwischen den Atomen 8 und 9 des R1-Restes), R2 für Methyl, R3 für Methyl, n für 3, m für 1, o für 1 und p für 1 steht. Explizit ganz besonders bevorzugt wird Cocamidopropyl Hydroxysultain eingesetzt, wie es beispielsweise unter dem Handelsnamen Mirataine CBS von der Firma Rhodia käuflich erhältlich ist.

Eine weitere ganz besonders bevorzugte Mehrkomponenten-Verpackungseinheit (Kit-of-parts) ist dadurch dadurch gekennzeichnet, dass das Mittel (c) in Container (C)
(c2) ein oder mehrere zwitterionische Tenside der Formel (II) enthält, 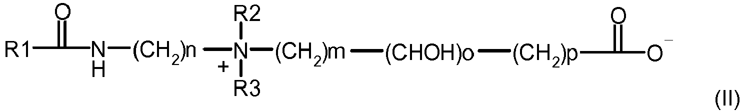 worin
   - R1: für eine lineare oder verzweigte C₉-C₂₉-Alkylgruppe oder eine lineare oder verzweigte, ein- oder mehrfach ungesättigte C₉-C₂₉-Alkenylgruppe steht,
   - R2, R3: jeweils unabhängig voneinander für eine C₁-C₆-Alkylgruppe stehen,
   - n: für eine ganze Zahl von 1 bis 6, bevorzugt für die Zahl 3, steht,
   - m: für eine ganze Zahl von 1 bis 6 steht,
   - o: für 0 steht, und
   - p: für 0 steht.

Ein Beispiel für besonders geeignete zwitterionische Tenside der Formel (II) ist Cocamidopropylbetain. Cocamidopropylbetain stellt ein Gemisch von Verbindungen der Formel (II) dar, bei denen R1 steht für C11-Alkyl bis C17-Alkyl, R2 für Methyl, R3 für Methyl, n gleich 3, m gleich 1, o gleich 0 und p gleich 0 steht.

Ein weiteres Beispiel für ein besonders geeignetes zwitterionisches Tenisd der Formel (II) ist Lauramidopropyl Betain, wobei R1 für C11-Alkyl steht, R2 für Methyl, R3 für Methyl, n gleich 3, m gleich 1, o gleich 0 und p gleich 0 steht.

Der Einsatz der erfindungsgemäßen, bevorzugten und besonders bevorzugten Tenside (c2) verhindert zusammen mit den Säuren aus der Gruppe (c1) die Nachdunklung der entfärbten Strähnen und sorgt auf diese Weise für einen lang anhaltenden Entfärbeeffekt. Von besonderem Vorteil ist hierbei der Einsatz der Tenside in bestimmten Mengenbereichen. Besonders bevorzugt ist es, wenn das Mittel (c) die zwitterionischen und/oder amphoteren Tenside (c2) in einer Gesamtmenge von 3,0 bis 50,0 Gew.-%, bevorzugt von 5,0 bis 40,0 Gew.-%, weiter bevorzugt von 7,0 bis 35,0 Gew.-% und besonders bevorzugt von 7,5 bis 25 Gew.-% - bezogen auf das Gesamtgewicht des Mittels (c) - enthält.

Eine ganz besonders bevorzugte Mehrkomponenten-Verpackungseinheit (Kit-of-parts) ist weiterhin dadurch dadurch gekennzeichnet, dass das Mittel (c) in Container (C)
(c2) ein oder mehrere zwitterionische und/oder amphotere Tenside in einer Gesamtmenge von 3,0 bis 50,0 Gew.-%, bevorzugt von 5,0 bis 40,0 Gew.-%, weiter bevorzugt von 7,0 bis 35,0 Gew.-% und besonders bevorzugt von 7,5 bis 25 Gew.-% - bezogen auf das Gesamtgewicht des Mittels (c) - enthält.

Eine weitere besonders bevorzugte Mehrkomponenten-Verpackungseinheit (Kit-of-parts) ist weiterhin dadurch dadurch gekennzeichnet, dass das Mittel (c) in Container (C)
(c2) ein oder mehrere zwitterionische Tenside in einer Gesamtmenge von 3,0 bis 50,0 Gew.-%, bevorzugt von 5,0 bis 40,0 Gew.-%, weiter bevorzugt von 7,0 bis 35,0 Gew.-% und besonders bevorzugt von 7,5 bis 25 Gew.-% - bezogen auf das Gesamtgewicht des Mittels (c) - enthält.

Eine weitere besonders bevorzugte Mehrkomponenten-Verpackungseinheit (Kit-of-parts) ist weiterhin dadurch dadurch gekennzeichnet, dass das Mittel (c) in Container (C)
(c2) ein oder mehrere amphotere Tenside in einer Gesamtmenge von 3,0 bis 50,0 Gew.-%, bevorzugt von 5,0 bis 40,0 Gew.-%, weiter bevorzugt von 7,0 bis 35,0 Gew.-% und besonders bevorzugt von 7,5 bis 25 Gew.-% - bezogen auf das Gesamtgewicht des Mittels (c) - enthält.

Wie zuvor beschrieben ist es von ganz besonderem Vorteil, wenn als Tenside (c2) zwitterionische Tenside eingesetzt werden. Insbesondere besonders bevorzugt ist es daher, wenn im Mittel (c) als Tenside (c2) zwitterionischen Tenside in einer Gesamtmenge von 3,0 bis 50,0 Gew.-%, bevorzugt von 5,0 bis 40,0 Gew.-%, weiter bevorzugt von 7,0 bis 35,0 Gew.-% und besonders bevorzugt von 7,5 bis 25 Gew.-% - bezogen auf das Gesamtgewicht des Mittels (c) - enthalten sind.

Aus diesem Grund ist auch die Mehrkomponenten-Verpackungseinheit (Kit-of-parts) zur reduktiven Entfärbung von gefärbten keratinischen Fasern ganz besonders geeignet, welche getrennt voneinander konfektioniert
(I) einen Container (A) enthaltend ein kosmetisches Mittel (a)
(II) einen Container (B) enthaltend ein kosmetisches Mittel (b) und
(III) einen Container (C) enthaltend ein kosmetisches, wässriges Mittel (c) umfasst,
wobei
- das Mittel (a) in Container (A)
   (a1) ein oder mehrere Reduktionsmittel aus der Gruppe Natriumdithionit, Zinkdithionit, Kaliumdithionit, Natriumsulfit, Natriumhydrogensulfit, Kaliumsulfit, Kaliumhydrogensulfit, Ammoniumsulfit, Natriumthiosulfat, Kaliumthiosulfat, Ammoniumthiosulfat, Hydroxymethansulfinsäure, Aminomethansulfinsäure, Cystein, Thiomilchsäure, Sulfanylessigsäure (Thioglycolsäure) und/oder Ascorbinsäure in einer Gesamtmenge von 0,5 bis 20,5 Gew.-%, bevorzugt von 3,5 bis 15,5 Gew.-%, weiter bevorzugt von 6,0 bis 13,5 Gew.-% und besonders bevorzugt von 7,5 bis 11,5 Gew.-% - bezogen auf das Gesamtgewicht des Mittels (a) - enthält,
- das Mittel (b) in Container (B)
   (b1) Wasser und
   (b2) ein oder mehrere Säuren aus der Gruppe Zitronensäure, Weinsäure, Äpfelsäure, Milchsäure Methansulfonsäure, Malonsäure, Oxalsäure und/oder 1-Hydroxyethan-1,1-diphosponsäure enthält,
- das Mittel (c) in Container (C)
   (c1) ein oder mehrere Säuren aus der Gruppe Zitronensäure, Weinsäure, Äpfelsäure, Milchsäure, Essigsäure, Hydroxyethan-1,1-diphosphonsäure, Methansulfonsäure, Benzosäure, Salzsäure, Schwefelsäure, Phosphorsäure, Malonsäure und/oder Oxalsäure in einer Gesamtmenge von 2,0 bis 20,0 Gew.-%, bevorzugt von 3,0 bis 18,0 Gew.-%, weiter bevorzugt von 4,0 bis 16,0 Gew.-% und besonders bevorzugt von 4,5 bis 14,0 Gew.-% - bezogen auf das Gesamtgewicht des Mittels (c) - enthält, und
   (c2) ein oder mehrere zwitterionische Tenside in einer Gesamtmenge von 3,0 bis 50,0 Gew.-%, bevorzugt von 5,0 bis 40,0 Gew.-%, weiter bevorzugt von 7,0 bis 35,0 Gew.-% und besonders bevorzugt von 7,5 bis 25 Gew.-% - bezogen auf das Gesamtgewicht des Mittels (c) - enthält

Das oder die zwitterionischen Tenside (c2) werden zusammen mit Säuren (c1) in einem wässrigen kosmetischen Träger eingesetzt und bilden so das erfindungsgemäße Nachbehandlungsmittel (c). Dieses Mittel (c) kann durch Zusatz von Säuren und Basen auf verschiedene pH-Werte eingestellt werden. Die Gruppierungen -COO⁻ -SO3⁻ der Tenside (c2) können in dem wässrigen kosmetischen Träger bei Veränderung des pH-Wertes in einer Gleichgewichtsreaktion auch teilweise protoniert werden. Auch diese durch die Gleichgewichtsreaktion protonierten Formen der zwitterionischen Tenside sind von dieser Erfindung mit umfasst. Alle angegebenen Mengenangaben beziehen sich auf die Menge der zwitterionischen Tenside, die in ihrer zwitterionischen Form dem wässrigen Träger hinzugefügt werden.

Auch das oder die amphoterenTenside (c2) werden zusammen mit Säuren (c1) in einem wässrigen kosmetischen Träger eingesetzt und bilden im Rahmen dieser Ausführungsform das erfindungsgemäße Nachbehandlungsmittel (c). Dieses Mittel (c) kann durch Zusatz von Säuren und Basen auf verschiedene pH-Werte eingestellt werden. Die Gruppierungen -COOH -SO3H der amphoteren Tenside (c2) können in dem wässrigen kosmetischen Träger bei Veränderung des pH-Wertes in einer Gleichgewichtsreaktion auch teilweise deprotoniert werden. Weiterhin können auch die im amphoteren Tensid vorhandenen protonierbaren Gruppierungen (wie beispielsweise primäre, sekundäre oder tertiätre Aminogruppen) abhängig von dem im Mittel (c) vorhandenen pH-Wert auch ganz oder teilweise in protonierter Form vorliegen. Alle diese Gleichgewichtsreaktionen, die die amphoteren Tenside bei Einstellung des pH-Wertes durchlaufen können, sind von dieser Erfindung ebenfalls mit umfasst. Alle angegebenen Mengenangaben beziehen sich auf die Menge der amphoteren Tenside, die in ihrer amphoteren Form (Säuregruppe protoniert, Aminogruppe nicht protoniert) dem wässrigen Träger hinzugefügt werden.

### Polyole

Es hat sich herausgestellt, dass der Einsatz von Polyolen die Entfärbewirkung weiter unterstützt. Aus diesem Grund ist es bevorzugt, wenn die erfindungsgemäßen Entärbemittel und/oder die erfindungsgemäßen Nachbehandlungsmittel zusätzlich ein odere mehrere Polyole enthalten. Unter einem Polyol wird eine Verbindung mit mindestens zwei aliphatischen (d.h. nicht phenolischen) OH-Gruppen verstanden.

Beispiele für geeignete erfindungsgemäße Polyole sind insbesondere Ethylengylcol, 1,2-Propylenglycol, 1,3-Propandiol, 1,2-Butandiol, 1,3-Butandiol, 1,2-Pentantiol, 1,3-Pentantiol, 1,4-Pentantiol, 1,5-Pentantiol, 1,2-Hexandiol, 1,3-hexandiol, 1,4-Hexantiol, 1,5-Hexandiol und 1,6-Hexandiol. Geeignet sind aber auch Polyethylenglycol und Polypropylenglycol.

In einer weiteren bevorzugten Ausführungsform ist eine erfindungsgemäße Mehrkomponenten-Verpackungseinheit (Kit-of-parts) daher dadurch gekennzeichnet, dass
- das Mittel (b) in Container (B) zusätzlich ein oder mehrere Polyole aus der Gruppe Ethylengylcol (1,2-Ethandiol), 1,2-Propandiol, 1,3-Propandiol, 1,2-Butandiol, 1,3-Butandiol, 1,4-Butandiol, 1,2-Pentantiol, 1,3-Pentantiol, 1,4-Pentantiol, 1,5-Pentantiol, 1,2-Hexandiol, 1,3-Hexandiol, 1,4-Hexantiol, 1,5-Hexandiol, 1,6-Hexandiol, Polyethylenglycol und/oder Polypropylenglycol enthält.

In einer weiteren bevorzugten Ausführungsform ist eine erfindungsgemäße Mehrkomponenten-Verpackungseinheit (Kit-of-parts) daher dadurch gekennzeichnet, dass
- das Mittel (c) in Container (C) zusätzlich ein oder mehrere Polyole aus der Gruppe Ethylengylcol (1,2-Ethandiol), 1,2-Propandiol, 1,3-Propandiol, 1,2-Butandiol, 1,3-Butandiol, 1,4-Butandiol, 1,2-Pentantiol, 1,3-Pentantiol, 1,4-Pentantiol, 1,5-Pentantiol, 1,2-Hexandiol, 1,3-Hexandiol, 1,4-Hexantiol, 1,5-Hexandiol, 1,6-Hexandiol, Polyethylenglycol und/oder Polypropylenglycol enthält. Die Polyole werden in den erfindungsgemäßen Mitteln (Mittel (b) und/oder Mittel (c)) bevorzugt in einer Gesamtmenge von 0,5 bis 15,0 Gew.-%, bevorzugt von 1,0 bis 11,5 Gew.-%, weiter bevorzugt von 1,5 bis 7,5 Gew.-% und besonders bevorzugt von 2,0 bis 4,5 Gew.-% - jeweils bezogen auf das Gesamtgewicht des Mittels, in dem das bzw. die Polyole jeweils eingesetzt werden - eingesetzt.

In einer weiteren bevorzugten Ausführungsform ist eine erfindungsgemäße Mehrkomponenten-Verpackungseinheit (Kit-of-parts) daher dadurch gekennzeichnet, dass
- das Mittel (b) in Container (B) zusätzlich ein oder mehrere Polyole aus der Gruppe Ethylengylcol (1,2-Ethandiol), 1,2-Propandiol, 1,3-Propandiol, 1,2-Butandiol, 1,3-Butandiol, 1,4-Butandiol, 1,2-Pentantiol, 1,3-Pentantiol, 1,4-Pentantiol, 1,5-Pentantiol, 1,2-Hexandiol, 1,3-Hexandiol, 1,4-Hexantiol, 1,5-Hexandiol, 1,6-Hexandiol, Polyethylenglycol und/oder Polypropylenglycol in einer Gesamtmenge von 0,5 bis 15,0 Gew.-%, bevorzugt von 1,0 bis 11,5 Gew.-%, weiter bevorzugt von 1,5 bis 7,5 Gew.-% und besonders bevorzugt von 2,0 bis 4,5 Gew.-% - bezogen auf das Gesamtgewicht des Mittels (a) - enthält.

In einer weiteren bevorzugten Ausführungsform ist eine erfindungsgemäße Mehrkomponenten-Verpackungseinheit (Kit-of-parts) daher dadurch gekennzeichnet, dass
- das Mittel (c) in Container (C) zusätzlich ein oder mehrere Polyole aus der Gruppe Ethylengylcol (1,2-Ethandiol), 1,2-Propandiol, 1,3-Propandiol, 1,2-Butandiol, 1,3-Butandiol, 1,4-Butandiol, 1,2-Pentantiol, 1,3-Pentantiol, 1,4-Pentantiol, 1,5-Pentantiol, 1,2-Hexandiol, 1,3-Hexandiol, 1,4-Hexantiol, 1,5-Hexandiol, 1,6-Hexandiol, Polyethylenglycol und/oder Polypropylenglycol in einer Gesamtmenge von 0,5 bis 15,0 Gew.-%, bevorzugt von 1,0 bis 11,5 Gew.-%, weiter bevorzugt von 1,5 bis 7,5 Gew.-% und besonders bevorzugt von 2,0 bis 4,5 Gew.-% - bezogen auf das Gesamtgewicht des Mittels (c) - enthält.

### Weitere Tenside

Die erfindungsgemäßen Nachbehandlungsmittel (c) können neben den erfindungswesentlichen amphoteren und/oder zwitterionischen Tensiden (b) auch noch weitere Tenside enthalten, wie beispielsweise nichtionische und/oder kationische Tenside. Es ist allerdings bevorzugt, wenn das Mittel (c) anionische Tenside nur in geringen Mengen enthält. In diesem Zusammenhang ist es ebenfalls bevorzugt, wenn auch die Mittel (a) und (b) die anionischen Tenside nur in geringen Mengen enthalten. Unter einer "geringen Menge" wird eine Menge von weniger als 1 Gew.-%, insbesondere eine Menge von maximal 0,9 Gew.-% verstanden, wobei die Angabe in Gewichtsprozent hierbei jeweils auf das Gesamtgewicht des jeweiligen Mittels (a), (b) und (c) bezogen ist.

In einer weiteren bevorzugten Ausführungsform ist eine erindungsgemäße Mehrkomponenten-Verpackungseinheit daher dadurch gekennzeichnet, dass
- die Gesamtmenge aller im Mittel (a) enthaltenen anionischen Tenside bei einem Wert von maximal 0,9 Gew.-%, bevorzugt von maximal 0,7 Gew.-%, weiter bevorzugt von maximal 0,5 Gew.-% und besonders bevorzugt von maximal 0,3 Gew.-% - bezogen auf das Gesamtgewicht des Mittels (a) - liegt,
- die Gesamtmenge aller im Mittel (b) enthaltenen anionischen Tenside bei einem Wert von maximal 0,9 Gew.-%, bevorzugt von maximal 0,7 Gew.-%, weiter bevorzugt von maximal 0,5 Gew.-% und besonders bevorzugt von maximal 0,3 Gew.-% - bezogen auf das Gesamtgewicht des Mittels (b) - liegt, und
- die Gesamtmenge aller im Mittel (c) enthaltenen anionischen Tenside bei einem Wert von maximal 0,9 Gew.-%, bevorzugt von maximal 0,7 Gew.-%, weiter bevorzugt von maximal 0,5 Gew.-% und besonders bevorzugt von maximal 0,3 Gew.-% - bezogen auf das Gesamtgewicht des Mittels (c) - liegt.

Unter anionischen Tensiden im Sinne dieser Erfindung werden Tenside mit ausschließlich anionischer Ladung verstanden.

Demnach ist es bevorzugt, wenn die Gesamtmenge aller jeweils in den Mitteln (a), (b) und (c) enthaltenen anionischen Tenside aus der Gruppe der
- linearen Fettsäuren mit 10 bis 22 C-Atomen (Seifen),
- Ethercarbonsäuren der Formel R-O-(CH2-CH2O)x -CH2-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionaten mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- linearen Alkansulfonaten mit 12 bis 18 C-Atomen,
- linearen Alpha-Olefinsulfonaten mit 12 bis 18 C-Atomen,
- Alpha-Sulfofettsäuremethylestern von Fettsäuren mit 12 bis 18 C-Atomen, und der
- Alkylsulfaten und Alkylpolyglykolethersulfaten der Formel R-O(CH2-CH2O)x-SO3H, in der R eine bevorzugt lineare Alkylgruppe mit 10 bis 18 C-Atomen und x = 0 oder 1 bis 12 ist,
bei einem Wert von maximal 0,9 Gew.-%, bevorzugt von maximal 0,7 Gew.-%, weiter bevorzugt von maximal 0,5 Gew.-% und besonders bevorzugt von maximal 0,3 Gew.-% - bezogen auf das Gesamtgewicht des Mittels - liegt.

Die Mittel (a), (b) und/oder (c) können weiterhin ein oder mehrere nichtionische Tenside enthalten. Unter nichtionischen Tensiden werden amphiphile (bifunktionelle) Verbindungen mit mindestens einem hydrophoben Rest und mindestens einem hydrophilen Molekülteil verstanden. Bei dem hydrophoben Molekülteil handelt es sich zumeist um eine Kohlenwasserstoffkette mit 10 bis 30 Kohlenstoff-Atomen. Als hydrophile Gruppe tragen die nichtionischen Tenside beispielsweise eine Polyolgruppe, eine Polyalkylenglykolethergruppe oder eine Kombination aus Polyol- und Polyglykolethergruppe. Solche Verbindungen sind beispielsweise Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in derAlkylgruppe, C12-C22-Fettsäuremono- und -diester von Anlagerungsprodukten von 1 bis 30 Mol Ethylenoxid an Glycerin, C8-C22-Alkylmono- und -oligoglycoside und deren ethoxylierte Analoga sowie Anlagerungsprodukte von 5 bis 60 Mol Ethylenoxid an Rizinusöl und gehärtetes Rizinusöl.

Die zusätzlich einsetzbaren nichtionischen Tenside können in den erfindungsgemäßen Mittel (a), (b) bzw.(c) jeweils in einer Gesamtmenge von 0,1 bis 15,0 Gew.-%, bevorzugt von 2,5 bis 13,5 Gew.-%, weiter bevorzugt von 3,5 bis 11,5 Gew.-% und besonders bevorzugt von 4,5 bis 9,5 Gew.-% - bezogen auf das Gesamtgewicht des jeweiligen Mittels - eingesetzt werden. Erfindungsgemäß werden als kationische Tenside insbesondere solche vom Typ der quartären Ammoniumverbindungen, der Esterquats und der Amidoamine eingesetzt. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche

Stearamidopropyldimethylamin dar. Weitere erfindungsgemäß verwendbare kationische Tenside stellen die quaternisierten Proteinhydrolysate
dar.

Die zusätzlich einsetzbaren kationischen Tenside können in den erfindungsgemäßen Mittel (a), (b) bzw. (c) jeweils in einer Gesamtmenge von von 0,1 bis 15,0 Gew.-%, bevorzugt von 2,5 bis 13,5 Gew.-%, weiter bevorzugt von 3,5 bis 11,5 Gew.-% und besonders bevorzugt von 4,5 bis 9,5 Gew.-% - bezogen auf das Gesamtgewicht des jeweiligen Mittels - eingesetzt werden.

### pH-Werte

Wie bereits zuvor beschrieben ist die Entfärbewirkung des anwendungsbereiten Entfärbemittels eine Funktion des pH-Wertes, die im sauren pH-Wertebereich ihr Optimum durchläuft. Bevorzugt besitzt das anwendungsbereite Entfärbemittel - d.h. die Mischung der Mittel (a) und (b) - daher einen sauren pH-Wert.

Aus Stabilitätsgründen ist es im Rahmen einer Ausführungsform bevorzugt, wenn das Mittel (a) wasserfrei konfektioniert ist.

Das Mittel (b) kann insbesondere auf einen pH-Wert von 1 bis 6, bevorzugt von 1,3 bis 4,5, weiter bevorzugt von 1,6 bis 4,0 und besonders bevorzugt von 2,0 bis 3,6 eingestellt werden.

Es hat sich herausgestellt, dass es auch von Vorteil ist, wenn auch das Entfärbemittel (c) sauer eingestellt ist, da eine Rückoxidation bzw. Nachdunklung in diesem Fall besonders gut verhindert wird.

Aus den vorgenannten Gründen ist eine ganz besonders bevorzugte Mehrkomponenten-Verpackungseinheit (Kit-of-parts) weiterhin dadurch dadurch gekennzeichnet, dass
- das Mittel (a) in Container (A) ein wasserfreies Mittel ist,
- das Mittel (b) in Container (B) ein wässriges Mittel ist, das einen pH-Wert von 1 bis 6, bevorzugt von 1,3 bis 4,5, weiter bevorzugt von 1,6 bis 4,0 und besonders bevorzugt von 2,0 bis 3,6 (gemessen mit einer Glaselektrode des Typs N61 der Firma Schott bei einer Temperatur von 22 °C) besitzt,
   und
- das Mittel (c) in Container (C) einen pH-Wert von 0,5 bis 4,0, bevorzugt von 0,7 bis 3,5, weiter bevorzugt von 0,9 bis 3,0 und besonders bevorzugt von 1,1 bis 2,5 (gemessen mit einer Glaselektrode des Typs N61 der Firma Schott bei einer Temperatur von 22 °C) besitzt.

Die pH-Werte wurden mit einer Glaselektrode des Typs N 61 der Firma Schott bei einer Temperatur von 22 °C gemessen.

"Wasserfrei" bedeutet in diesem Zusammenhang, dass das Mittel (a) einen Wassergehalt von höchstens 10,0 Gew.-%, bevorzugt von höchstens 5,0 Gew.-%, weiter bevorzugt von höchstens 2,5 Gew.-%, noch weiter bevorzugt von höchstens 1,0 Gew.-% und am allermeisten bevorzugt von höchstens 0,5 Gew.-% - bezogen auf das Gesamtgewicht des Mittels (a) - besitzt.

Mit anderen Worten ist eine eine ganz besonders bevorzugte Mehrkomponenten-Verpackungseinheit (Kit-of-parts) weiterhin dadurch dadurch gekennzeichnet, dass
- das Mittel (a) in Container (A) einen Wassergehalt von höchstens 10,0 Gew.-%, bevorzugt von höchstens 5,0 Gew.-%, weiter bevorzugt von höchstens 2,5 Gew.-%, noch weiter bevorzugt von höchstens 1,0 Gew.-% und am meisten bevorzugt von höchstens 0,5 Gew.-% - bezogen auf das Gesamtgewicht des Mittels (a) - besitzt,
- das Mittel (b) in Container (B) ein wässriges Mittel ist, das einen pH-Wert von 1 bis 6, bevorzugt von 1,3 bis 4,5, weiter bevorzugt von 1,6 bis 4,0 und besonders bevorzugt von 2,0 bis 3,6 (gemessen mit einer Glaselektrode des Typs N61 der Firma Schott bei einer Temperatur von 22 °C) besitzt,
   und
- das Mittel (c) in Container (C) einen pH-Wert von 0,5 bis 4,0, bevorzugt von 0,7 bis 3,5, weiter bevorzugt von 0,9 bis 3,0 und besonders bevorzugt von 1,1 bis 2,5 (gemessen mit einer Glaselektrode des Typs N61 der Firma Schott bei einer Temperatur von 22 °C) besitzt.

Obwohl der pH-Wert der Mittel (b) und (c) bevorzugt im sauren Bereich liegt, können die Mittel zur Feineinstellung des pH-Wertes dennoch geringe Mengen an Alkalisierungsmitteln enthalten. Die zu diesem Zweck erfindungsgemäß verwendbaren Alkalisierungsmittel können aus der Gruppe, die gebildet wird aus Ammoniak, Alkanolaminen, basischen Aminosäuren, sowie anorganischen Alkalisierungsmitteln wie (Erd-)Alkalimetallhydroxiden, (Erd-)Alkalimetallmetasilikaten, (Erd-)-Alkalimetallphosphaten und (Erd-)Alkalimetallhydrogenphosphaten, ausgewählt werden. Geeignete anorganische Alkalisierungsmittel sind Natriumhydroxid, Kaliumhydroxid, Natriumsilicat und Natriummetasilicat. Erfindungsgemäß einsetzbare, organische Alkalisierungsmittel können ausgewählt werden aus Monoethanolamin, 2-Amino-2-methylpropanol und Triethanolamin. Die als erfindungsgemäßes Alkalisierungsmittel einsetzbaren basischen Aminosäuren können ausgewählt werden aus der Gruppe, die gebildet wird aus Arginin, Lysin, Ornithin und Histidin.

### Entfärbung gefärbter Keratinfasern

Bei der erfindungsgemäßen Mehrkomponenten-Verpackungseinheit handelt es sich um ein die Mittel (a), (b) und (c) umfassendes System, dass zur Entfärbung von zuvor gefärbten keratinischen Fasern, insbesondere menschlichen Haaren, eingesetzt wird. Bei den gefärbten Keratinfasern handelt es sich üblicherweise um Fasern, die zuvor mit herkömmlichen, dem Fachmann bekannten Oxidationsfarbstoffen und/oder direktziehenden Farbstoffen gefärbt wurden.

Die Entfärbemittel sind geeignet zur Entfernung von Färbungen, die mit Oxidationsfarbstoffen auf Basis von Entwickler- und Kupplerkomonenten auf den Keratinfasern erzeugt wurden. Wenn als Entwickler die folgenden Verbindungen einsetzt wurden, lassen sich die hiermit erzeugten Färbungen durch Einsatz des Entfärbemittel gut, effektiv und nahezu ohne spätere Nachdunklung entfernen: p-Phenylendiamin, p-ToluylendiaminN,N-Bis-(β-hydroxyethyl)-p-phenylendiamin, 4-N,N-Bis-(β-hydroxyethyl)-amino-2-methylanilin, 2-(β-Hydroxyethyl)-p-phenylendiamin, 2-(α,β-Dihydroxyethyl)-p-phenylendiamin, 2-Hydroxymethyl-p-phenylendiamin, Bis-(2-hydroxy-5-aminophenyl)-methan, p-Aminophenol, 4-Amino-3-methylphenol, 2,4,5,6-Tetraaminopyrimidin, 4-Hydroxy-2,5,6-triaminopyrimidin, 2-Hydroxy-4,5,6-triaminopyrimidin und/oder 4,5-Diamino-1-(β-hydroxyethyl)-pyrazol.

Wenn als Kuppler die folgenden Verbindungen einsetzt wurden, lassen sich die hiermit erzeugten Färbungen ebenfalls mit sehr gutem Entfärbeergebnis entfernen: m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2',4'-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.

1-Naphthol, 1,5-Dihydroxynaphthalin, 2,7-Dihydroxynaphthalin, 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Das zu entfärbende Substrat kann ebenso mit direktziehenden Farbstoffen eingefärbt worden sein. Als direktziehende Farbstoffe kommen dabei insbesondere Nitrophenylendiamine, Nitroaminophenole, Azofarbstoffe, Anthrachinone oder Indophenole in Frage. Mit der erfindungsgemäßen Mehrkomponenten-Verpackungseinheit (Kit-of-parts) lassen sich beispielweise Keratinfasern entfärben, die mit den unter den internationalen Bezeichnungen bzw. Handelsnamen bekannten Farbstoffen gefärbt wurden: HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Weiterhin können die zu entfärbenden Substrate auch mit in der Natur vorkommenden, natürlichen Farbstoffen, wie sie beispielsweise in Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten sind, gefärbt sein.

Die erfindungsgmäßen Entfärbemittel sind zur Entfernung dieser Färbungen gedacht und enhalten selbst daher bevorzugt keine Farbstoffe, d.h. keine Oxidationsfarbstoffvorprodukte vom Entwicklertyp und vom Kupplertyp und auch keine direktziehenden Farbstoffe.

In einer weiteren bevorzugten Ausführungsform ist ein erfindungsgemäße Mehrkomponenten-Verpackungseinheit (Kit-of-parts) deshalb dadurch gekennzeichnet, dass
- die Gesamtmenge aller im Mittel (a) enthaltenen Farbstoffe und Oxidationsfarbstoffvorprodukte bei einem Wert von maximal 0,2 Gew.-%, bevorzugt von maximal 0,1 Gew.-%, weiter bevorzugt von maximal 0,05 Gew.-% und besonders bevorzugt von maximal 0,01 Gew.-% - bezogen auf das Gesamtgewicht des Mittels (a) - liegt,
- die Gesamtmenge aller im Mittel (b) enthaltenen Farbstoffe und Oxidationsfarbstoffvorprodukte bei einem Wert von maximal 0,2 Gew.-%, bevorzugt von maximal 0,1 Gew.-%, weiter bevorzugt von maximal 0,05 Gew.-% und besonders bevorzugt von maximal 0,01 Gew.-% - bezogen auf das Gesamtgewicht des Mittels (b) - liegt, und
- die Gesamtmenge aller im Mittel (c) enthaltenen Farbstoffe und Oxidationsfarbstoffvorprodukte bei einem Wert von maximal 0,2 Gew.-%, bevorzugt von maximal 0,1 Gew.-%, weiter bevorzugt von maximal 0,05 Gew.-% und besonders bevorzugt von maximal 0,01 Gew.-% - bezogen auf das Gesamtgewicht des Mittels (c) - liegt.

### Oxidationsmittel

Die erfindungsgemäßen Mehrkomponenten-Verpackungseinheit wird zur reduktiven Entfärbung von gefärbten keratinischen Fasern eingesetzt. Die Mittel (a) und (b) bilden hierbei zusammen das anwendungsbereite Entfärbemittel, welches ein Reduktionsmittel enthält. Aus Gründen der Inkompabilität und zur Vermeidung von exothermen, unkontrollierbaren Reaktionen enthalten die Mittel (a) und (b) daher vorzugsweise kein Oxidationsmittel.

Die sukzessive Behandlung von Keratinfasern mit Reduktionsmitteln und Oxidationsmitteln kann zu sehr starken Haarschädigungen führen. Um die auftretenden Schäden an den Keratinfasern so gering wie möglich zu halten, ist es daher zudem bevorzugt, wenn auch das Nachbehandlungsmittel (c) kein Oxidationsmittel enthält.

Unter Oxidationsmitteln werden hierbei insbesondere die auch zur oxidativen Entfärbung einsetzbaren Oxidationsmittel wie beispielsweise Wasserstoffperoxid und Persulfate (Kaliumpersulfat (alternativ Kaliumperoxodisulfat), Natriumpersulfat (Natriumperoxodisulfat) und Ammoniumpersulfat (alternativ Ammoniumperoxodisulfat)) verstanden. Bevorzugt enthält daher keines der Mittel (a), (b) und (c) die vorgenannten Oxidationsmittel.

In einer weiteren bevorzugten Ausführungsform ist ein erfindungsgemäße Mehrkomponenten-Verpackungseinheit (Kit-of-parts) deshalb dadurch gekennzeichnet, dass
- die Gesamtmenge aller im Mittel (a) enthaltenen Oxidationsmittel aus der Gruppe der Peroxide und der Persulfate bei einem Wert von maximal 0,2 Gew.-%, bevorzugt von maximal 0,1 Gew.-%, weiter bevorzugt von maximal 0,05 Gew.-% und besonders bevorzugt von maximal 0,01 Gew.-% - bezogen auf das Gesamtgewicht des Mittels (a) - liegt,
- die Gesamtmenge aller im Mittel (b) enthaltenen Oxidationsmittel aus der Gruppe der Peroxide und der Persulfate bei einem Wert von maximal 0,2 Gew.-%, bevorzugt von maximal 0,1 Gew.-%, weiter bevorzugt von maximal 0,05 Gew.-% und besonders bevorzugt von maximal 0,01 Gew.-% - bezogen auf das Gesamtgewicht des Mittels (b) - liegt,
- die Gesamtmenge aller im Mittel (c) enthaltenen Oxidationsmittel aus der Gruppe der Peroxide und der Persulfate bei einem Wert von maximal 0,2 Gew.-%, bevorzugt von maximal 0,1 Gew.-%, weiter bevorzugt von maximal 0,05 Gew.-% und besonders bevorzugt von maximal 0,01 Gew.-% - bezogen auf das Gesamtgewicht des Mittels (c) - liegt.

### Mischungsverhältnis der Mittel (a) und (b)

Wie bereits zuvor beschrieben wird das anwendungsbereite Entfärbemittel durch Vermischen der Mittel (a) und (b) hergestellt. Prinzipiell können die Mittel (a) und (b) hierbei in verschiedenen Mischungsverhältnissen, wie beispielsweise (a)/(b) von 20:1 bis 1:20, vermischt werden.

Um eine komfortable Vermischung zu gewährleisten, kann von Vorteil sein, die beiden Mittel (a) und (b) in ungefähr gleichen Mengen einzusetzen. Insbesondere, wenn im Mittel (b) Säuren in konzentrierterer Form eingesetzt werden, kann es auch von Vorteil sein, das Mittel (a) in einem Überschuss einzusetzen. Wird das Mittel (a) jedoch wasserfrei formuliert, so kann es andererseits auch von Vorteil sein, das Mittel (b) in einem Überschuss einzusetzen.

Das Mittel (a) enthalt das bzw. die Reduktionsmittel (a1) bevorzugt in einer Gesamtmenge von 25,0 bis 100 Gew.-%, bevorzugt von 45,0 bis 100 Gew.-%, weiter bevorzugt von 65,0 bis 100 Gew.-% und besonders bevorzugt von 85,0 bis 100 Gew.-% - bezogen auf das Gesamtgewicht des Mittels (a). Im Mittel (a) liegt das bzw. liegen die Reduktionsmittel daher verhältnismäßig hochkonzentriert vor.

Gerade wenn die Reduktionsmittel in diesen auch in diesen Konzentrationsbereichen im Mittel (a) eingesetzt werden, ist der Einsatz eines Überschusses an Mittel (b) wünschenswert.

In einer weiteren bevorzugten Ausführungsform ist eine erfindungsgemäße Mehrkomponenten-Verpackungseinheit daher dadurch gekennzeichnet, dass die Mengen des Mittels (a) in Container (A) und des Mittels (b) in Container (B) so gewählt werden, dass bei Herstellung der Anwendungsmischung - d.h. bei Vermischung der Mittel (a) und (b) - das Mischungsverhältnis (a)/(b) bei einem Wert von 1:5 bis 1:30 liegt.

In einer weiteren besonders bevorzugten Ausführungsform ist eine erfindungsgemäße Mehrkomponenten- Verpackungseinheit daher dadurch gekennzeichnet, dass die Mengen des Mittels (a) in Container (A) und des Mittels (b) in Container (B) so gewählt werden, dass bei Herstellung der Anwendungsmischung - d.h. bei Vermischung der Mittel (a) und (b) - das Mischungsverhältnis (a)/(b) bei einem Wert von 1:8 bis 1:20, ganz besonders bevorzugt bei einem Wert von 1:10 bis 1:15, liegt.

Zur Herstellung der Mischung kann beispielsweise das Mittel (a) aus Container (A) vollständig in Container (B) - der bereits das Mittel (b) enthält - überführt werden. In diesem Fall wird die Größe des Containers (B) so gewählt, dass der Container (B) die Gesamtmenge der Mittel (a) und (b) aufnehmen kann und auch ein Vermischen der beiden Mittel (a) und (b), z.B. durch Verschütteln oder Verrühren, zulässt.

Analog kann die Herstellung der Mischung auch durch vollständige Überführung des Mittels (b) aus Container (B) in Container (A) - der bereits das Mittel (a) enthält - erfolgen. In diesem Fall sollte die Größe des Containers (A) so gewählt werden, dass der Container (A) die Gesamtmenge der Mittel (a) und (b) aufnehmen kann und auch ein Vermischen der beiden Mittel (a) und (b), z.B. durch Verschütteln, oder Verrühren, zulässt.

Eine weitere Möglichkeit zur Herstellung der Anwendungsmischung ist die vollständige Überführung beider Mittel (a) und (b) aus den Containern (A) und (B) in ein drittes Behältnis, welches dann das Vermischen beider Mittel - z.B. durch Verschütteln, oder Verrühren - erlaubt.

Beispiel: Eine erfindungsgemäße Mehrkompenenten-Verpackungseinheit enthält
- 10 g Natriumdithionit, Mittel (a) in Container (A) (d.h. der Gesamtgehalt an Reduktionsmittel(n) (a1) in Mittel (a) beträgt 100 Gew.-% (abzüglich geringer Verunreinigungen in Natriumditionit)
- 100 g des Mittels (b) in Conatiner (B)

Zur Herstellung der Anwendungsmischung wird das Mittel (a) aus Container (A) vollständig in Container (B) überführt. Die Mittel (a) und (b) werden dann miteinander verschüttelt oder verrührt. Das Mischungsverhältnis der Mittel (a)/(b) liegt bei einem Wert von (10 g / 100 g) = 1:10.

### Weitere Inhaltsstoffe

Ferner können die erfindungsgemäßen Mittel (a), (b) und/oder (c) weitere Wirk-, Hilfs- und Zusatzstoffe enthalten, wie beispielsweise nichtionische Polymere wie beispielsweise Vinylpyrrolidinon/Vinylacrylat-Copolymere, Polyvinylpyrrolidinon, Vinylpyrrolidinon/VinylacetatCopolymere, Polyethylenglykole und Polysiloxane; zusätzliche Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Polyoxyalkylen(B)-Blockcopolymere, gepfropften Silikonpolymere; kationische Polymere wie quaternisierte Celluloseether, Polysiloxane mit quaternären Gruppen, Dimethyldiallylammoniumchlorid-Polymere, Acrylamid-Dimethyldiallyl-ammoniumchlorid-Copolymere, mit Diethylsulfat quaternierte Dimethylamino-ethylmethacrylat-Vinylpyrrolidinon-Copolymere, Vinylpyrrolidinon-Imidazolinium-methochlorid-Copolymere und quaternierter Polyvinylalkohol; zwitterionische und amphotere Polymere; anionische Polymere wie beispielsweise Polyacrylsäuren oder vernetzte Polyacrylsäuren; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; Pigmente sowie Treibmittel wie Propan-Butan-Gemische, N₂O, Dimethylether, CO₂ und Luft. Es wird in diesem Zusammenhang ausdrücklich auf die bekannten Monographien, z. B. Kh. Schrader, Grundlagen und Rezepturen der Kosmetika, 2. Auflage, Hüthig Buch Verlag, Heidelberg, 1989, verwiesen, die das entsprechende Wissen des Fachmannes wiedergeben.

### Verfahren

Die erfindungsgemäßen, zuvor beschriebenen Mehrkomponenten-Verpackungseinheiten (Kit-of-Parts) lassen sich in Verfahren zur reduktiven Entfärbung einsetzen.

Ein zweiter Gegenstand der vorliegenden Erfindung ist daher ein Verfahren zur reduktiven Entfärbung von gefärbten keratinischen Fasern, umfassend die folgenden Schritte in der angegebenen Reihenfolge
(I) Herstellung eines anwendungsbereiten Entfärbemittels durch Vermischen eines Mittels (a), wie es bei Beschreibung des ersten Erfindungsgegenstands definiert wurde, mit einem Mittel (b), wie es bei Beschreibung des ersten Erfindungsgegenstands definiert wurde,
(II) Applizierung des anwendungsbereiten Entfärbemittels auf keratinischen Fasern,
(III) Einwirkenlassen des Entfärbemittels für einen Zeitraum von 5 bis 60 Minuten, bevorzugt von 10 bis 55 Minuten, weiter bevorzugt von 20 bis 50 Minuten und besonders bevorzugt von 30 bis 45 Minuten,
(IV) Abspülen des Entfärbemittels von den keratinischen Fasern,
(V) Applizierung eines kosmetischen wässrigen Mittels (c), wie es bei Beschreibung des ersten Erfindungsgegenstands definiert wurde, auf die keratinischen Fasern,
(VI) Einwirkenlassen des Mittels (c) für einen Zeitraum von 30 Sekunden bis 30 Minuten, bevorzugt von 30 Sekunden bis 20 Minuten, weiter bevorzugt von 30 Sekunden bis 10 Minuten und besonders bevorzugt von 30 Sekunden bis 5 Minuten, und
(VII) Abspülen des Mittels (c) von den keratinischen Fasern.

Die Schritte (I), (II), (III) und (IV) des Verfahrens stellen den Entfärbevorgang der Keratinfasern dar und werden demzufolge in direkter zeitlicher Abfolge hintereinander ausgeführt. Für die Abfolge der Schritte (IV) und (V) besteht prinzipiell keine zeitliche Limitierung. So kann Schritt (V) Stunden, Tage oder beispielsweise auch bis zu zwei Wochen nach Abschluss des Schrittes (IV) erfolgen. Das Verfahren soll jedoch die Rückdunklung bzw. Rückoxidation verhindern, die durch Einwirkung von Luftsauerstoff auf die entfärbten Keratinfasern, auftreten kann,. Um diese Rückoxidation wirkungsvoll zu verhindern, sollte die Nachbehandlung stattfinden, bevor der Luftsauerstoff über einen zu langen Zeitraum auf die reduzierten Keratinfasern einwirken kann. Aus diesem Grund sollte die Nachbehandlung nach Möglichkeit direkt im Anschluss an die Entfärbung (d.h. zeitlich direkt nach Abschluss des Verfahrensschrittes (IV)) stattfinden. Aus diesem Grund ist es bevorzugt, wenn zwischen dem Abschluss des Verfahrensschrittes (IV) und dem Beginn des Vefahrensschrittes (V) ein Zeitraum von maximal 12 Stunden, bevorzugt von maximal 6 Stunden, weiter bevorzugt von maximal 1 Stunde und besonders bevorzugt von maximal 30 Minuten liegt. Ein bevorzugtes erfindungsgemäßes Verfahren ist somit dadurch gekennzeichnet, dass zwischen den Verfahrensschritten (IV) und (V) ein Zeitraum von maximal 12 Stunden, bevorzugt von maximal 6 Stunden, weiter bevorzugt von maximal 1 Stunde und besonders bevorzugt von maximal 30 Minuten liegt.

Die Anwendung des Nachbehandlungsmittels kann auch mehrmals wiederholt werden, beispielsweise, wenn es sich bei dem Mittel (c) um ein Shampoo handelt, welches regelmäßig nach der Entfärbung angewendet wird. Wird die Nachbehandlung, d.h. die Durchführung der Schritte (V) bis (VII) wiederholt, so wird es möglich, die Rückoxidation für einen besonders langen Zeitraum zu unterdrücken.

Ein besonders bevorzugtes erfindungsgemäßes Verfahren ist daher dadurch gekennzeichnet, dass im Anschluss an Schritt (VII) die folgenden Schritte in der angegebenen Reihenfolge durchgeführt werden
(VIII) Applizierung eines kosmetischen wässrigen Mittels (c) auf die keratinischen Fasern, wobei das Mittel (c) ein Mittel ist, wie es bei Beschreibung des ersten Erfindungsgegenstands definiert wurde,
(IX) Einwirkenlassen des Mittels (c) für einen Zeitraum von 30 Sekunden bis 30 Minuten, bevorzugt von 30 Sekunden bis 20 Minuten, weiter bevorzugt von 30 Sekunden bis 10 Minuten und besonders bevorzugt von 30 Sekunden bis 5 Minuten, und
(X) Abspülen des Mittels (c) von den keratinischen Fasern.

Die Schritte (VIII) bis (X) stellen eine Wiederholung der Nachbehandlung mit dem Mittel (c) dar; diese Nachbehandlung erfolgt im Anschluss an Schritt (VII). Für die Abfolge der Schritte (VII) und (VIII) besteht prinzipiell keine zeitliche Limitierung. Aus Komfortabilitätsgründen für den Anwender ist es jedoch bevorzugt wenn die Nachbehandlung mit dem Mittel (c) im Rahmen der üblichen, vom Anwender durchgeführten Haarwäsche durchgeführt wird. Wäscht der Anwender sich beispielsweise normalerweise alle ein bis zwei Tage die Haare, so sollte für diese Haarwäsche das Nachbehandlungsmittel (c) verwendet werden. Demzufolge ist es bevorzugt, wenn zwischen den Schritten (VII) und (VIII) ein zeitlicher Abstand von 12 bis 48 Stunden, bevorzugt von 24 ist 36 Stunden liegt.

Das erfindungsgemäße Verfahren ist insbesondere wirkungsvoll bei Keratinfasern, die mit bestimmten Oxidationsfarbstoffvorprodukten gefärbt wurden.

Gute Ergebnisse wurden vor allem erzielt, wenn das Entfärbeverfahren auf Keratinfasern angewendet wurde, die mit einem oder mehreren Oxidationsfarbstoffvorprodukten aus der Gruppe p-Phenylendiamin, p-Toluylendiamin, N,N-Bis-(β-hydroxyethyl)-p-phenylendiamin, 2-(β-Hydroxyethyl)-p-phenylendiamin und/oder 2-(Methoxymethyl)-p-phenylendiamin gefärbt wurden. Ein bevorzugtes erfindungsgemäßes Verfahren ist daher weiterhin dadurch gekennzeichnet, dass das anwendungsbereite Entfärbemittel auf keratinische Fasern appliziert wird, die mit mindestens einem Oxidationsfarbstoffvorprodukt aus der Gruppe p-Phenylendiamin, p-Toluylendiamin, N,N-Bis-(β-hydroxyethyl)-p-phenylendiamin, 2-(β-Hydroxyethyl)-p-phenylendiamin und/oder 2-(Methoxymethyl)-p-phenylendiamin gefärbt wurden.

Bezüglich weiterer bevorzugter Ausführungsformen der erfindungsgemäßen Verfahrens gilt mutatis mutandis das zu dem erfindungsgemäßen Mitteln Gesagte.

### Beispiele

### 1.1. Färbung

Es wurden die folgenden Formulierungen hergestellt (alle Angaben in Gew.-%): Färbecreme (F1)

| Rohstoff | Gew.% |
|---|---|
| Cetearylalkohol | 8,5 |
| C12-C18 Fettalkohole | 3,0 |
| Ceteareth-20 | 0,5 |
| Ceteareth-12 | 0,5 |
| Plantacare 1200 UP (Laurylglucoside, 50 - 53 %ige wässrige Lösung) | 2,0 |
| Natrium Laureth-6 Carboxylat (21 %ige wässrige Lösung) | 10,0 |
| Natriummyreth Sulfat (68 - 73 %ige wässrige Lösung) | 2,8 |
| Sodium acrylate, trimethylammoniopropylacrylamide chloride copolymer (19 - 21 %ige wässrige Lösung) | 3,8 |
| Kaliumhydroxid | 0,83 |
| p-Toluylendiamin, Sulfat | 2,25 |
| m-Aminophenol | 0,075 |
| 2-Am i no-3-hyd roxypyrid in | 0,12 |
| Resorcin | 0,62 |
| 4-Chlorresorcin | 0,26 |
| 3-Amino-2-methylamino-6-methoxypyridin | 0,04 |
| 1,3-Bis(2,4-diaminophenoxy)propan, Tetrahydrochlorid | 0,05 |
| Ammoniumsulfat | 0,1 |
| Natriumsulfit | 0,4 |
| Ascorbinsäure | 0,1 |
| 1-Hydroxyethan-1,1-diphosphonsäure (60 %ige wässrige Lösung) | 0,2 |
| Ammoniak (25 %ige wässrige Lösung) | 7,2 |
| Wasser | Ad 100 |

### Oxidationsmittel (Ox)

| Rohstoff | Gew.% |
|---|---|
| Natriumbenzoat | 0,04 |
| Dipicolinsäure | 0,1 |
| Dinatriumpyrophosphat | 0,1 |
| Kaliumhydroxid | 0,09 |
| 1,2-Propylenglycol | 1,0 |
| 1-Hydroxyethan-1,1-diphosphonsäure (60 %ige wässrige Lösung) | 0,25 |
| Paraffinum Liquidum | 0,30 |
| Steartrimoniumchlorid | 0,39 |
| Cetearylalkohol | 3,4 |
| Ceteareth-20 | 1,0 |
| Wasserstoffperoxid (50 %ige wässrige Lösung) | 12,0 |

Die Farbcreme (F1) und das Oxidationsmittel (Ox) wurden im Mengenverhältnis 1:1 gemischt und auf Haarsträhnen (Kerling Euronaturhaar weiß) appliziert. Das Gewichtsverhältnis Anwendungsmischung : Haar betrug 4:1, die Einwirkzeit betrug 30 Minuten bei einer Temperatur von 32 Grad Celsius. Anschließend wurden die Strähnen mit Wasser gespült, getrocknet und mindestens 24 Stunden bei Raumtemperatur ruhen gelassen. Die Strähnen wurden in einem dunkelbraunen Farbton gefärbt.

### 1.2. Entfärbung

Es wurden die folgenden Entfärbemittel hergestellt (alle Angaben in Gew.-% Aktivsubstanz):
Mittel (a)

| Mittel (a) | Gew.-% |
|---|---|
| Natriumditionit | 8,8 |

Mittel (b)

| Mittel (b) | Gew.-% |
|---|---|
| Cetearylalkohol | 2,9 |
| PEG-40 Castor Oil | 0,55 |
| Natriumcetearylsulfat | 0,28 |
| Hydroxyethan-1,1-disposphonsäure (1-Etidronsäure) | 0,24 |
| Wasser (dest.) | ad 100 |
| pH-Wert | 2,0 |

Mittel (c)

| Mittel (c) | (C1) | (C2) | (C3) | (C4) |
|---|---|---|---|---|
| Cocamidopropylbetain | 11,4 | 11,4 | --- | --- |
| Cocamidopropyl Hydroxysultain | -- | -- | 12,5 | 12,5 |
| Oxalsäure | 4,5 | 4,5 | 4,5 | 4,5 |
| Methansulfonsäure | --- | 2,1 | --- | 2,1 |
| PEG-12 Dimethicone | 1,6 | 1,6 | 1,6 | 1,6 |
| Xanthan | 1,3 | 1,3 | 1,3 | 1,3 |
| Propylenglycol | 2,6 | 2,6 | 2,6 | 2,6 |
| Wasser | ad 100 | ad 100 | ad 100 | ad 100 |
| pH-Wert | 2,0 - 2,4 | 2,0 - 2,4 | 2,0 - 2,4 | 2,0 - 2,4 |

Die Mittel (a) und (b) wurden vermischt (8,8 g des Mittels (a) wurden mit 100 g des Mittels (b) vermischt). Dieses anwendungsbereite Entfärbemittel wurde auf die unter Punkt 1.1 colorierten Haare aufgetragen und für 45 Minuten bei einer Temperatur von 30 °C einwirken gelassen. Danach wurden die Strähnen für 20 Sekunden mit Wasser ausgespült.

Direkt im Anschluss daran wurden die entfärbten Strähnen für 1 Minute mit dem Mittel (c) behandelt und wieder für 20 Sekunden mit Wasser ausgespült. Die Behandlung mit dem Mittel (c) wurde noch zweimal wiederholt. Dann wurden die Strähen gründlich für 5 Minuten mit Wasser ausgewaschen und getrocknet.

Die Färbung der Strähnen wurde in Abhängigkeit von der Zeit visuell beurteilt. Die Bewertung der Farbintensität erfolgte anhand der folgenden Skala:
0 - Strähne besitzt keine wahrnehmbare Färbung mehr (weiß-blond, wie die ursprüngliche Färbung des eingesetzten Kerling Euronaturhaar weiß)
1 - Strähne mit schwacher Farbintensität gefärbt
2 - Strähne mit mittlerer Farbintensität gefärbt
3 - Strähne mit starker Farbintensität gefärbt
4 - Färbung der Strähne wie direkt nach der Färbung, kein Entfärbe-Effekt

Als Referenz (Versuch V, Vergleich) diente eine Strähne, die wie zuvor unter Punkt 1.1. beschrieben gefärbt wurde. Dann wurden die Mittel (a) und (b) vermischt (8,8 g des Mittels (a) wurden wieder mit 100 g des Mittels (b) vermischt). Dieses anwendungsbereite Entfärbemittel wurde auf die colorierten Haare aufgetragen und für 45 Minuten bei einer Temperatur von 30 °C einwirken gelassen. Danach wurden die Strähnen für 5 Minuten mit Wasser ausgespült.

Die Referenzsträhne wurde demnach mit dem anwendungsbereiten Entfärbemittel ((a) + (b)), nicht jedoch mit dem Nachbehandlungsmittel (c) behandelt. Nach der Entfärbung (nach der Anwendung des Mittels (a) + (b)) wurde die Strähne lediglich gründlich für einen Zeitraum von 5 Minuten mit Wasser ausgewaschen.

Die Färbung der Referenzsträhne (Versuch V, Vergleich) wurde ebenfalls in Abhängigkeit von der Zeit beobachtet.

Es wurden die folgenden Ergebnisse erhalten:

| | V | (C1) | (C2) | (C3) | (C4) |
|---|---|---|---|---|---|
| Färbung der Strähne während des Auswaschens (5 Minuten) | 2 - 3 | 1 - 2 | 0 - 1 | 1 - 2 | 0 - 1 |
| Färbung der Strähne nach 10 Minuten | 3 | 2 | 1 | 2 | 1 |
| Färbung der Strähne nach 60 Minuten | 3 | 2 | 1 | 2 | 1 |
| Färbung der Strähne nach 1 Tag | 3-4 | 2 | 1 | 2 | 1 |

## Patentansprüche

1. Mehrkomponenten-Verpackungseinheit (Kit-of-parts) zur reduktiven Entfärbung von gefärbten keratinischen Fasern, welche getrennt voneinander konfektioniert
(I) einen Container (A) enthaltend ein kosmetisches Mittel (a)
(II) einen Container (B) enthaltend ein kosmetisches Mittel (b) und
(III) einen Container (C) enthaltend ein kosmetisches, wässriges Mittel (c) umfasst,
wobei
- das Mittel (a) in Container (A)
(a1) ein oder mehrere Reduktionsmittel aus der Gruppe Natriumdithionit, Zinkdithionit, Kaliumdithionit, Natriumsulfit, Natriumhydrogensulfit, Kaliumsulfit, Kaliumhydrogensulfit, Ammoniumsulfit, Natriumthiosulfat, Kaliumthiosulfat, Ammoniumthiosulfat, Hydroxymethansulfinsäure, Aminomethansulfinsäure, Cystein, Thiomilchsäure, Sulfanylessigsäure (Thioglycolsäure) und/oder Ascorbinsäure enthält, und
- das Mittel (c) in Container (C)
(c1) ein oder mehrere Säuren aus der Gruppe anorganischen und/oder
organischen Säuren und (c2) ein oder mehrere zwitterionische und/oder amphotere Tenside enthält.

2. Mehrkomponenten-Verpackungseinheit (Kit-of-parts) nach Anspruch 1, **dadurch gekennzeichnet dass** das Mittel (a) in Container (A)
(a1) ein oder mehrere Reduktionsmittel aus der Gruppe Natriumdithionit, Zinkdithionit, Kaliumdithionit, Natriumsulfit, Natriumhydrogensulfit, Kaliumsulfit, Kaliumhydrogensulfit, Ammoniumsulfit, Natriumthiosulfat, Kaliumthiosulfat und/oder Ammoniumthiosulfat in einer Gesamtmenge von 25,0 bis 100 Gew.-%, bevorzugt von 45,0 bis 100 Gew.-%, weiter bevorzugt von 65,0 bis 100 Gew.-% und besonders bevorzugt von 85,0 bis 100 Gew.-% - bezogen auf das Gesamtgewicht des Mittels (a) - enthält.

3. Mehrkomponenten-Verpackungseinheit (Kit-of-parts) nach einem der Ansprüche 1 bis 2, **dadurch gekennzeichnet, dass** das Mittel (b) in Container (B)
(b1) Wasser und
(b2) ein oder mehrere Säuren aus der Gruppe anorganischen und/oder organischen Säuren enthält.

4. Mehrkomponenten-Verpackungseinheit (Kit-of-parts) nach einem der Ansprüche 1 bis 3, **dadurch gekennzeichnet dass** das Mittel (c) in Container (C)
(c1) ein oder mehrere Säuren aus der Gruppe Zitronensäure, Weinsäure, Äpfelsäure, Milchsäure, Essigsäure, Hydroxyethan-1,1-diphosphonsäure, Methansulfonsäure, Benzosäure, Salzsäure, Schwefelsäure, Phosphorsäure, Malonsäure und/oder Oxalsäure in einer Gesamtmenge von 2,0 bis 20,0 Gew.-%, bevorzugt von 3,0 bis 18,0 Gew.-%, weiter bevorzugt von 3,5 bis 16,0 Gew.-% und besonders bevorzugt von 4,0 bis 14,0 Gew.-% - bezogen auf das Gesamtgewicht des Mittels (c) - enthält.

5. Mehrkomponenten-Verpackungseinheit (Kit-of-parts) nach einem der Ansprüche 1 bis 4, **dadurch gekennzeichnet, dass** das Mittel (c) in Container (C)
(c1) als Säuren Oxalsäure und/oder Malonsäure sowie eine weitere Säure aus der Gruppe
Zitronensäure, Weinsäure, Äpfelsäure, Milchsäure, Essigsäure, Hydroxyethan-1,1-diphosphonsäure, Methansulfonsäure, Benzosäure, Salzsäure, Schwefelsäure, Phosphorsäure enthält.

6. Mehrkomponenten-Verpackungseinheit (Kit-of-parts) nach einem der Ansprüche 1 bis 5, **dadurch gekennzeichnet, dass** das Mittel (c) in Container (C)
(c2) ein oder mehrere zwitterionische Tenside ausgewählt aus der Gruppe der Formeln (I) bis (IV) enthält, 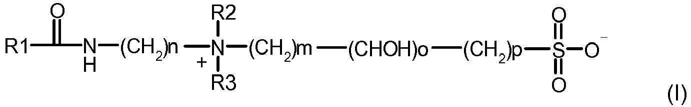 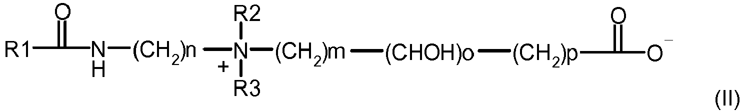 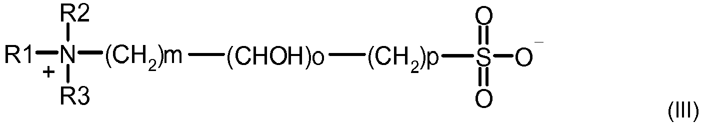 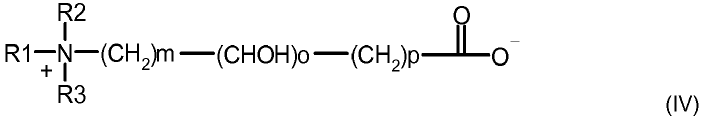 worin
R1 jeweils unabhängig voneinander für eine lineare oder verzweigte C₉-C₂₉-Alkylgruppe, eine lineare oder verzweigte C₉-C₂₉-Alkenylgruppe oder eine lineare oder verzweigte Hydroxy-C₉-C₂₉-Alkylgruppe steht,
R2, R3 jeweils unabhängig voneinander für eine C₁-C₆-Alkylgruppe, eine C₂-C₆-Alkenylgruppe oder eine Hydroxy-C₂-C₆-alkylgruppe stehen,
n jeweils unabhängig voneinander für eine ganze Zahl von 1 bis 6 steht,
m jeweils unabhängig voneinander für eine ganze Zahl von 0 bis 6 steht,
o jeweils unabhängig voneinander für eine ganze Zahl von 0 bis 6 steht,
p jeweils unabhängig voneinander für eine ganze Zahl von 0 bis 6 steht,
mit der Maßgabe, das die Summe aus m, o und p jeweils mindestens 1 beträgt.

7. Mehrkomponenten-Verpackungseinheit (Kit-of-parts) nach einem der Ansprüche 1 bis 6, **dadurch gekennzeichnet, dass** das Mittel (c) in Container (C)
(c2) ein oder mehrere zwitterionische Tenside der Formel (I) enthält, 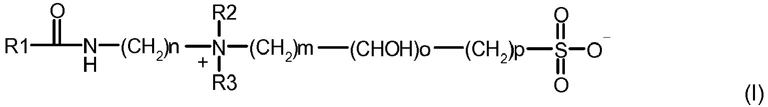 worin
R1 für eine lineare oder verzweigte C₉-C₂₉-Alkylgruppe oder eine lineare oder verzweigte, ein- oder mehrfach ungesättigte C₉-C₂₉-Alkenylgruppe steht,
R2, R3 jeweils unabhängig voneinander für eine C₁-C₆-Alkylgruppe stehen,
n für eine ganze Zahl von 1 bis 6, bevorzugt für die Zahl 3, steht,
m für eine ganze Zahl von 1 bis 6 steht,
o für eine ganze Zahl von 1 bis 6 steht, und
p für eine ganze Zahl von 1 bis 6 steht.

8. Mehrkomponenten-Verpackungseinheit (Kit-of-parts) nach einem der Ansprüche 1 bis 7, **dadurch gekennzeichnet, dass** das Mittel (c) in Container (C)
(c2) ein oder mehrere zwitterionische Tenside der Formel (II) enthält, 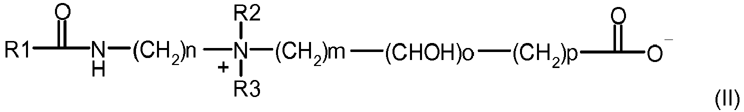 worin
R1 für eine lineare oder verzweigte C₉-C₂₉-Alkylgruppe oder eine lineare oder verzweigte, ein- oder mehrfach ungesättigte C₉-C₂₉-Alkenylgruppe steht,
R2, R3 jeweils unabhängig voneinander für eine C₁-C₆-Alkylgruppe stehen,
n für eine ganze Zahl von 1 bis 6, bevorzugt für die Zahl 3, steht,
m für eine ganze Zahl von 1 bis 6 steht,
o für 0 steht, und
p für 0 steht.

9. Mehrkomponenten-Verpackungseinheit (Kit-of-parts) nach einem der Ansprüche 1 bis 8, **dadurch gekennzeichnet, dass** dass das Mittel (c) in Container (C)
(c2) ein oder mehrere zwitterionische und/oder amphotere Tenside in einer Gesamtmenge von 3,0 bis 50,0 Gew.-%, bevorzugt von 5,0 bis 40,0 Gew.-%, weiter bevorzugt von 7,0 bis 35,0 Gew.-% und besonders bevorzugt von 7,5 bis 25 Gew.-% - bezogen auf das Gesamtgewicht des Mittels (c) - enthält.

10. Mehrkomponenten-Verpackungseinheit (Kit-of-parts) nach einem der Ansprüche 1 bis 9, **dadurch gekennzeichnet, dass**
- das Mittel (a) in Container (A) ein wasserfreies Mittel ist,
- das Mittel (b) in Container (B) ein wässriges Mittel ist, das einen pH-Wert von 1 bis 6, bevorzugt von 1,3 bis 4,5, weiter bevorzugt von 1,6 bis 4,0 und besonders bevorzugt von 2,0 bis 3,6 besitzt, und
- das Mittel (c) in Container (C) einen pH-Wert von 0,5 bis 4,0, bevorzugt von 0,7 bis 3,5, weiter bevorzugt von 0,9 bis 3,0 und besonders bevorzugt von 1,1 bis 2,5 besitzt.

11. Mehrkomponenten-Verpackungseinheit (Kit-of-parts) nach einem der Ansprüche 1 bis 10, **dadurch gekennzeichnet, dass**
- die Gesamtmenge aller im Mittel (a) enthaltenen Farbstoffe und Oxidationsfarbstoffvorprodukte bei einem Wert von maximal 0,2 Gew.-%, bevorzugt von maximal 0,1 Gew.-%, weiter bevorzugt von maximal 0,05 Gew.-% und besonders bevorzugt von maximal 0,01 Gew.-% - bezogen auf das Gesamtgewicht des Mittels (a) - liegt,
- die Gesamtmenge aller im Mittel (b) enthaltenen Farbstoffe und Oxidationsfarbstoffvorprodukte bei einem Wert von maximal 0,2 Gew.-%, bevorzugt von maximal 0,1 Gew.-%, weiter bevorzugt von maximal 0,05 Gew.-% und besonders bevorzugt von maximal 0,01 Gew.-% - bezogen auf das Gesamtgewicht des Mittels (b) - liegt, und
- die Gesamtmenge aller im Mittel (c) enthaltenen Farbstoffe und Oxidationsfarbstoffvorprodukte bei einem Wert von maximal 0,2 Gew.-%, bevorzugt von maximal 0,1 Gew.-%, weiter bevorzugt von maximal 0,05 Gew.-% und besonders bevorzugt von maximal 0,01 Gew.-% - bezogen auf das Gesamtgewicht des Mittels (c) - liegt.

12. Mehrkomponenten-Verpackungseinheit (Kit-of-parts) nach einem der Ansprüche 1 bis 11, **dadurch gekennzeichnet, dass**
- die Gesamtmenge aller im Mittel (a) enthaltenen Oxidationsmittel aus der Gruppe der Peroxide und der Persulfate bei einem Wert von maximal 0,2 Gew.-%, bevorzugt von maximal 0,1 Gew.-%, weiter bevorzugt von maximal 0,05 Gew.-% und besonders bevorzugt von maximal 0,01 Gew.-% - bezogen auf das Gesamtgewicht des Mittels (a) - liegt,
- die Gesamtmenge aller im Mittel (b) enthaltenen Oxidationsmittel aus der Gruppe der Peroxide und der Persulfate bei einem Wert von maximal 0,2 Gew.-%, bevorzugt von maximal 0,1 Gew.-%, weiter bevorzugt von maximal 0,05 Gew.-% und besonders bevorzugt von maximal 0,01 Gew.-% - bezogen auf das Gesamtgewicht des Mittels (b) - liegt, und
- die Gesamtmenge aller im Mittel (c) enthaltenen Oxidationsmittel aus der Gruppe der Peroxide und der Persulfate bei einem Wert von maximal 0,2 Gew.-%, bevorzugt von maximal 0,1 Gew.-%, weiter bevorzugt von maximal 0,05 Gew.-% und besonders bevorzugt von maximal 0,01 Gew.-% - bezogen auf das Gesamtgewicht des Mittels (c) - liegt.

13. Verfahren zur reduktiven Entfärbung von gefärbten keratinischen Fasern, umfassend die folgenden Schritte in der angegebenen Reihenfolge
(I) Herstellung eines anwendungsbereiten Entfärbemittels durch Vermischen eines Mittels (a), wie es in den Ansprüchen 1, 2, 10, 11 und/oder 12 definiert wurde, mit einem Mittel (b), wie es in den Ansprüchen 1, 3, 10, 11 und/oder 12 definiert wurde,
(II) Applizierung des anwendungsbereiten Entfärbemittels auf keratinische Fasern,
(III) Einwirkenlassen des Entfärbemittels für einen Zeitraum von 5 bis 60 Minuten, bevorzugt von 10 bis 55 Minuten, weiter bevorzugt von 20 bis 50 Minuten und besonders bevorzugt von 30 bis 45 Minuten,
(IV) Abspülen des Entfärbemittels von den keratinischen Fasern,
(V) Applizierung eines kosmetischen wässrigen Mittels (c) auf die keratinischen Fasern, wobei das Mittel (c) ein Mittel ist, wie es in den Ansprüchen 1, 4, 5, 6, 7, 8, 9, 10, 11 und/oder 12 definiert wurde,
(VI) Einwirkenlassen des Mittels (c) für einen Zeitraum von 30 Sekunden bis 30 Minuten, bevorzugt von 30 Sekunden bis 20 Minuten, weiter bevorzugt von 30 Sekunden bis 10 Minuten und besonders bevorzugt von 30 Sekunden bis 5 Minuten, und
(VII) Abspülen des Mittels (c) von den keratinischen Fasern.

14. Verfahren nach Anspruch 13, **dadurch gekennzeichnet, dass** im Anschluss an Schritt (VII) die folgenden Schritte in der angegebenen Reihenfolge durchgeführt werden (VIII) Applizierung eines kosmetischen wässrigen Mittels (c) auf die keratinischen Fasern, wobei das Mittel (c) ein Mittel ist, wie es in den Ansprüchen 1, 4, 5, 6, 7, 8, 9, 10, 11 und/oder 12 definiert wurde,
(IX) Einwirkenlassen des Mittels (c) für einen Zeitraum von 30 Sekunden bis 30 Minuten, bevorzugt von 30 Sekunden bis 20 Minuten, weiter bevorzugt von 30 Sekunden bis 10 Minuten und besonders bevorzugt von 30 Sekunden bis 5 Minuten, und
(X) Abspülen des Mittels (c) von den keratinischen Fasern.

15. Verfahren nach einem der Ansprüche 13 bis 14, **dadurch gekennzeichnet, dass** das anwendungsbereite Entfärbemittel auf keratinische Fasern appliziert wird, die mit mindestens einem Oxidationsfarbstoffvorprodukt aus der Gruppe p-Phenylendiamin, p-Toluylendiamin, N,N-Bis-(β-hydroxyethyl)-p-phenylendiamin, 2-(β-Hydroxyethyl)-p-phenylendiamin und/oder 2-(Methoxymethyl)-p-phenylendiamin gefärbt wurden.

## Claims

1. A multi-component packaging unit (kit-of-parts) for reductive decolorization of colored keratin fibers, which unit comprises, packaged separately from one another:
(I) a container (A) containing a cosmetic agent (a)
(II) a container (B) containing a cosmetic agent (b) and
(III) a container (C) containing a cosmetic, aqueous agent (c),
wherein
- the agent (a) in container (A) contains
(a1) one or more reduction agents from the group of sodium dithionite, zinc dithionite, potassium dithionite, sodium sulfite, sodium hydrogen sulfite, potassium sulfite, potassium hydrogen sulfite, ammonium sulfite, sodium thiosulfate, potassium thiosulfate, ammonium thiosulfate, hydroxymethanesulfinic acid, aminomethanesulfinic acid, cysteine, thiolactic acid, sulfanyl acetic acid (thioglycolic acid) and/or ascorbic acid, and
- the agent (c) in container (C) contains
(c1) one or more acids from the group of inorganic and/or organic acids and
(c2) one or more zwitterionic and/or amphoteric surfactants.

2. The multi-component packaging unit (kit-of-parts) according to claim 1, **characterized in that** the agent (a) in container (A) contains
(a1) one or more reduction agents from the group of sodium dithionite, zinc dithionite, potassium dithionite, sodium sulfite, sodium hydrogen sulfite, potassium sulfite, potassium hydrogen sulfite, ammonium sulfite, sodium thiosulfate, potassium thiosulfate and/or ammonium thiosulfate in a total amount of from 25.0 to 100 wt.%, preferably 45.0 to 100 wt.%, more preferably 65.0 to 100 wt.% and particularly preferably 85.0 to 100 wt.%, based on the total weight of the agent (a).

3. The multi-component packaging unit (kit-of-parts) according to one of claims 1 to 2, **characterized in that** the agent (b) in container (B) contains
(b1) water and
(b2) one or more acids from the group of inorganic and/or organic acids.

4. The multi-component packaging unit (kit-of-parts) according to one of claims 1 to 3, **characterized in that** the agent (c) in container (C) contains
(c1) one or more acids from the group of citric acid, tartaric acid, malic acid, lactic acid, acetic acid, hydroxyethane-1,1-diphosphonic acid, methane sulfonic acid, benzoic acid, hydrochloric acid, sulfuric acid, phosphoric acid, malonic acid and/or oxalic acid in a total amount of from 2.0 to 20.0 wt.%, preferably 3.0 to 18.0 wt.%, more preferably 3.5 to 16.0 wt.% and particularly preferably 4.0 to 14.0 wt.%, based on the total weight of the agent (c).

5. The multi-component packaging unit (kit-of-parts) according to one of claims 1 to 4, **characterized in that** the agent (c) in container (C) contains
(c1) as acids, oxalic acid and/or malonic acid and a further acid from the group citric acid, tartaric acid, malic acid, lactic acid, acetic acid, hydroxyethane-1,1-diphosphonic acid, methane sulfonic acid, benzoic acid, hydrochloric acid, sulfuric acid and phosphoric acid.

6. The multi-component packaging unit (kit-of-parts) according to one of claims 1 to 5, **characterized in that** the agent (c) in container (C) contains
(c2) one or more zwitterionic surfactants selected from the group of formulas (I) to (IV), 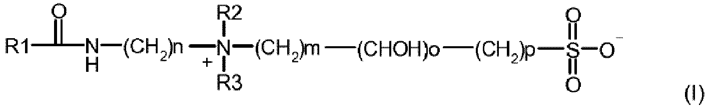 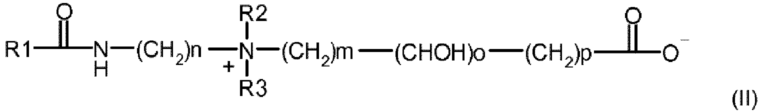 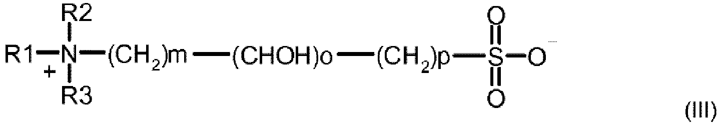 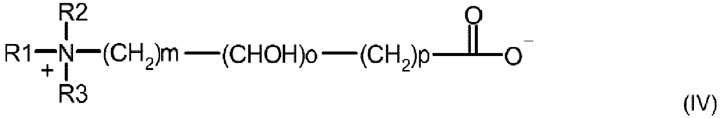 where
R1 in each case independently represents a linear or branched C₉-C₂₉ alkyl group, a linear or branched C₉-C₂₉ alkenyl group or a linear or branched hydroxy C₉-C₂₉ alkyl group,
R2, R3 in each case independently represent a C₁-C₆ alkyl group, a C₂-C₆ alkenyl group or a hydroxy C₂-C₆ alkyl group,
n in each case independently represents an integer from 1 to 6,
m in each case independently represents an integer from 0 to 6,
o in each case independently represents an integer from 0 to 6,
p in each case independently represents an integer from 0 to 6, with the proviso that the sum total of m, o and p is in each case at least 1.

7. The multi-component packaging unit (kit-of-parts) according to one of claims 1 to 6, **characterized in that** the agent (c) in container (C) contains
(c2) one or more zwitterionic surfactants of formula (I), 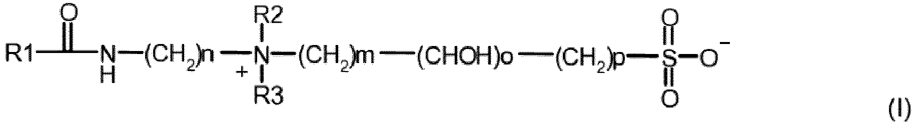 where
R1 represents a linear or branched C₉-C₂₉ alkyl group or a linear or branched, mono or polyunsaturated C₉-C₂₉ alkenyl group,
R2, R3 in each case independently represent a C₁-C₆ alkyl group,
n represents an integer from 1 to 6, preferably 3,
m represents an integer from 1 to 6,
o represents an integer from 1 to 6, and
p represents an integer from 1 to 6.

8. The multi-component packaging unit (kit-of-parts) according to one of claims 1 to 7, **characterized in that** the agent (c) in container (C) contains
(c2) one or more zwitterionic surfactants of formula (II), 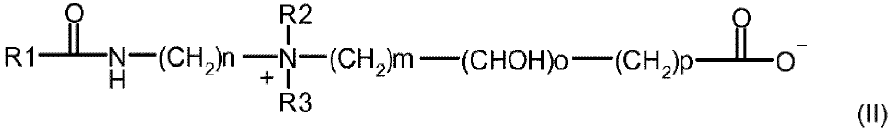 where
R1 represents a linear or branched C₉-C₂₉ alkyl group or a linear or branched, mono or polyunsaturated C₉-C₂₉ alkenyl group,
R2, R3 in each case independently represent a C₁-C₆ alkyl group,
n represents an integer from 1 to 6, preferably 3,
m represents an integer from 1 to 6,
o represents 0, and
p represents 0.

9. The multi-component packaging unit (kit-of-parts) according to one of claims 1 to 8, **characterized in that** the agent (c) in container (C) contains
(c2) one or more zwitterionic and/or amphoteric surfactants in a total amount of from 3.0 to 50.0 wt.%, preferably 5.0 to 40.0 wt.%, more preferably 7.0 to 35.0 wt.% and particularly preferably 7.5 to 25 wt.%, based on the total weight of the agent (c).

10. The multi-component packaging unit (kit-of-parts) according to one of claims 1 to 9, **characterized in that**
- the agent (a) in container (A) is a water-free agent,
- the agent (b) in container (B) is an aqueous agent that has a pH of from 1 to 6, preferably 1.3 to 4.5, more preferably 1.6 to 4.0 and particularly preferably 2.0 to 3.6, and
- the agent (c) in container (C) has a pH of 0.5 to 4.0, preferably 0.7 to 3.5, more preferably 0.9 to 3.0 and particularly preferably 1.1 to 2.5.

11. The multi-component packaging unit (kit-of-parts) according to one of claims 1 to 10, **characterized in that**
- the total amount of all dyes contained in the agent (a) and oxidation dye precursors is at a value of at most 0.2 wt.%, preferably at most 0.1 wt.%, more preferably at most 0.05 wt.% and particularly preferably at most 0.01 wt.%, based on the total weight of the agent (a),
- the total amount of all dyes contained in the agent (b) and oxidation dye precursors is at a value of at most 0.2 wt.%, preferably at most 0.1 wt.%, more preferably at most 0.05 wt.% and particularly preferably at most 0.01 wt.%, based on the total weight of the agent (b), and
- the total amount of all dyes contained in the agent (c) and oxidation dye precursors is at a value of at most 0.2 wt.%, preferably at most 0.1 wt.%, more preferably at most 0.05 wt.% and particularly preferably at most 0.01 wt.%, based on the total weight of the agent (c).

12. The multi-component packaging unit (kit-of-parts) according to one of claims 1 to 11, **characterized in that**
- the total amount of all oxidants from the group of peroxides and persulfates contained in the agent (a) is at a value of at most 0.2 wt.%, preferably at most 0.1 wt.%, more preferably at most 0.05 wt.% and particularly preferably at most 0.01 wt.%, based on the total weight of the agent (a),
- the total amount of all oxidants from the group of peroxides and persulfates contained in the agent (b) is at a value of at most 0.2 wt.%, preferably at most 0.1 wt.%, more preferably at most 0.05 wt.% and particularly preferably at most 0.01 wt.%, based on the total weight of the agent (b), and
- the total amount of all oxidants from the group of peroxides and persulfates contained in the agent (c) is at a value of at most 0.2 wt.%, preferably at most 0.1 wt.%, more preferably at most 0.05 wt.% and particularly preferably at most 0.01 wt.%, based on the total weight of the agent (c).

13. A method for reductive decolorization of colored keratin fibers, comprising the following steps in the order indicated
(I) producing a ready-to-use decoloring agent by mixing an agent (a) as defined in claims 1, 2, 10, 11 and/or 12 with an agent (b) as defined in claims 1,3, 10, 11 and/or 12,
(II) applying the ready-to-use decoloring agent to keratin fibers,
(III) leaving the decoloring agent to act for a period of 5 to 60 minutes, preferably 10 to 55 minutes, more preferably 20 to 50 minutes and particularly preferably 30 to 45 minutes,
(IV) rinsing the decoloring agent off the keratin fibers,
(V) applying a cosmetic aqueous agent (c) to the keratin fibers, wherein the agent (c) is an agent as defined in claims 1, 4, 5, 6, 7, 8, 9, 10, 11 and/or 12,
(VI) leaving the agent (c) to act for a period of 30 seconds to 30 minutes, preferably 30 seconds to 20 minutes, more preferably 30 seconds to 10 minutes and particularly preferably 30 seconds to 5 minutes, and
(VII) rinsing the agent (c) off the keratin fibers.

14. The method according to claim 13, **characterized in that**, subsequent to step (VII), the following steps are carried out in the order indicated
(VII) applying a cosmetic aqueous agent (c) to the keratin fibers, the agent (c) being an agent as defined in claims 1, 4, 5, 6, 7, 8, 9, 10, 11 and/or 12,
(IX) leaving the agent (c) to act for a period of 30 seconds to 30 minutes, preferably 30 seconds to 20 minutes, more preferably 30 seconds to 10 minutes and particularly preferably 30 seconds to 5 minutes, and
(X) rinsing the agent (c) off the keratin fibers.

15. The method according to one of claims 13 to 14, **characterized in that** the ready-to-use decoloring agent is applied to keratin fibers which have been dyed with at least one oxidation dye precursor from the group p-phenylene diamine, p-toluenediamine, N,N-bis-(β-hydroxyethyl-p-phenylene diamine, 2-(β-hydroxyethyl)-p-phenylene diamine and/or 2-(methoxymethyl)-p-phenylene diamine.

## Revendications

1. Unité de conditionnement multi-composante (*kit of parts*) destinée à la décoloration réductrice de fibres de kératine colorées, contenant, préparés séparément les uns des autres
(I) un récipient (A) contenant un agent cosmétique (a)
(II) un récipient (B) contenant un agent cosmétique (b) et
(III) un récipient (C) contenant un agent cosmétique aqueux (c),
où
- l'agent (a) dans le récipient (A) contient
(a1) au moins un réducteur issu du groupe constitué de dithionite de sodium, de dithionite de zinc, de dithionite de potassium, de sulfite de sodium, d'hydrogénosulfite de sodium, de sulfite de potassium, d'hydrogénosulfite de potassium, de sulfite d'ammonium, de thiosulfate de sodium, de thiosulfate de potassium, de thiosulfate d'ammonium, d'acide hydroxyméthansulfinique, d'acide aminométhansulfinique, de cystéine, d'acide thiolactique, d'acide sulfanylacétique (acide thioglycolique) et/ou d'acide ascorbique, et
- l'agent (c) dans le récipient (C) contient
(c1) au moins un acide issu du groupe des acides minéraux et/ou organiques, et
(c2) au moins un tensioactif zwitterionique et/ou amphotère.

2. Unité de conditionnement multi-composante (*kit of parts*) selon la revendication 1, **caractérisée en ce que** l'agent (a) dans le récipient (A) contient
(a1) au moins un réducteur issu du groupe constitué de dithionite de sodium, dithionite de zinc, dithionite de potassium, sulfite de sodium, hydrogénosulfite de sodium, sulfite de potassium, hydrogénosulfite de potassium, sulfite d'ammonium, thiosulfate de sodium, thiosulfate de potassium et/ou thiosulfate d'ammonium à hauteur d'un total de 25,0 à 100 % en poids, de préférence de 45,0 à 100 % en poids, plus préférentiellement de 65,0 à 100 % en poids et particulièrement préférentiellement de 85,0 à 100 % en poids - rapporté au poids total de l'agent (a).

3. Unité de conditionnement multi-composante (*kit of parts*) selon une des revendications 1 à 2, **caractérisée en ce que** l'agent (b) dans le récipient (B) contient
(b1) de l'eau, et
(b2) au moins un acide issu du groupe des acides minéraux et/ou organiques.

4. Unité de conditionnement multi-composante (*kit of parts*) selon une des revendications 1 à 3, **caractérisée en ce que** l'agent (c) dans le récipient (C)
(c1) au moins un acide issu du groupe constitué de l'acide citrique, l'acide tartrique, l'acide malique, l'acide lactique, l'acide acétique, l'acide hydroxyéthan-1,1-diphosphonique, l'acide méthanesulfonique, l'acide benzoïque, l'acide chlorhydrique, l'acide sulfurique, l'acide phosphorique, l'acide malonique et/ou l'acide oxalique à hauteur d'un total de 2,0 à 20,0 % en poids, de préférence de 3,0 à 18,0 % en poids, plus préférentiellement de 3,5 à 16,0 % en poids et particulièrement préférentiellement de 4,0 à 14,0 % en poids - rapporté au poids total de l'agent (c).

5. Unité de conditionnement multi-composante (*kit of parts*) selon une des revendications 1 à 4, **caractérisée en ce que** l'agent (c) dans le récipient (C) contient
(c1) comme acides l'acide oxalique et/ou l'acide malonique, ainsi qu'un autre acide issu du groupe constitué de l'acide citrique, l'acide tartrique, l'acide malique, l'acide lactique, l'acide acétique, l'acide hydroxyéthan-1,1-diphosphonique, l'acide méthanesulfonique, l'acide benzoïque, l'acide chlorhydrique, l'acide sulfurique, l'acide phosphorique.

6. Unité de conditionnement multi-composante (*kit of parts*) selon une des revendications 1 à 5, **caractérisée en ce que** l'agent (c) dans le récipient (C) contient
(c2) au moins un tensioactif zwitterionique choisi dans le groupe selon les formules (I) à (IV) 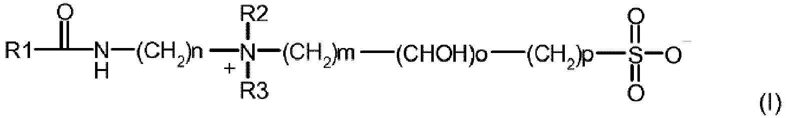 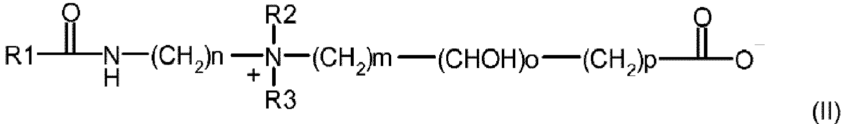 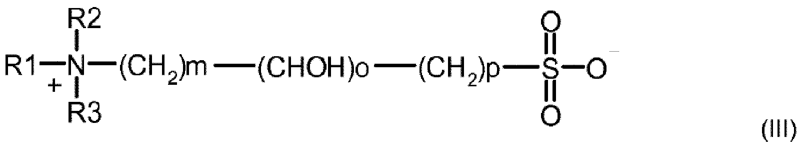 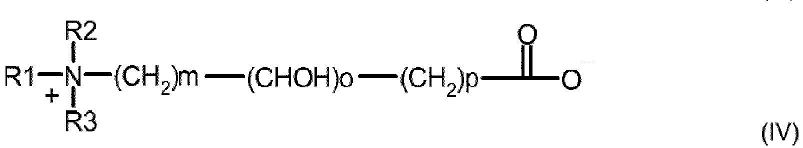 où
R1 représente respectivement, indépendamment les uns des autres, un groupe C₉₋₂₉-alkyle linéaire ou ramifié, un groupe C₉₋₂₉-alcényle linéaire ou ramifié ou un groupe hydroxy-C₉₋₂₉-alkyle linéaire ou ramifié.
R2, R3 représentent respectivement, indépendamment les uns des autres, un groupe C₁₋₆-alkyle, un groupe C₂₋₆-alcényle ou un groupe hydroxy-C₂₋₆-alkyle,
n représente respectivement, indépendamment les uns des autres, un entier de 1 à 6,
m représente respectivement, indépendamment les uns des autres, un entier de 0 à 6,
o représente respectivement, indépendamment les uns des autres, un entier de 0 à 6,
p représente respectivement, indépendamment les uns des autres, un entier de 0 à 6, à condition que la somme m+o+p ≥ 1.

7. Unité de conditionnement multi-composante (*kit of parts*) selon une des revendications 1 à 6, **caractérisée en ce que** l'agent (c) dans le récipient (C) contient
(c2) au moins un tensioactif zwitterionique de formule (I) 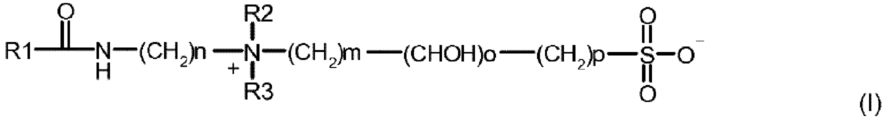 où
R1 représente respectivement, indépendamment les uns des autres, un groupe C₉₋₂₉-alkyle linéaire ou ramifié, un groupe C₉₋₂₉-alcényle linéaire ou ramifié, mono- ou polyinsaturé,
R2, R3 représentent respectivement, indépendamment les uns des autres, un groupe C₁₋₆-alkyle,
n représente un entier de 1 à 6, de préférence 3,
m représente un entier de 1 à 6,
o représente un entier de 1 à 6, et
p représente un entier de 1 à 6.

8. Unité de conditionnement multi-composante (*kit of parts*) selon une des revendications 1 à 7, **caractérisée en ce que** l'agent (c) dans le récipient (C) contient
(c2) au moins un tensioactif zwitterionique de formule (II) 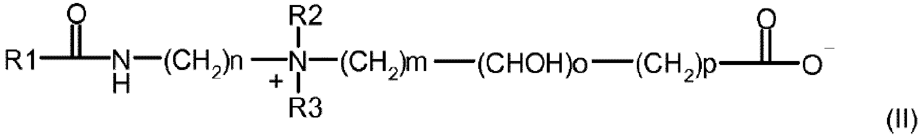 où
R1 représente respectivement, indépendamment les uns des autres, un groupe C₉₋₂₉-alkyle linéaire ou ramifié, un groupe C₉₋₂₉-alcényle linéaire ou ramifié, mono- ou polyinsaturé, R2, R3 représente respectivement, indépendamment les uns des autres, un groupe C₁₋₆-alkyle,
n représente un entier de 1 à 6, de préférence 3,
m représente un entier de 1 à 6,
o = 0,
p = 0.

9. Unité de conditionnement multi-composante (*kit of parts*) selon une des revendications 1 à 8, **caractérisée en ce que** l'agent (c) dans le récipient (C) contient
(c2) au moins un tensioactif zwitterionique et/ou amphotère à hauteur d'un total de 3,0 à 50,0 % en poids, de préférence de 5,0 à 40,0 % en poids, plus préférentiellement de 7,0 à 35,0 % en poids et particulièrement préférentiellement de 7,5 à 25 % en poids - rapporté au poids total de l'agent (c).

10. Unité de conditionnement multi-composante (*kit of parts*) selon une des revendications 1 à 9, **caractérisée en ce que**
- l'agent (a) dans le récipient (A) est un agent anhydre,
- l'agent (b) dans le récipient (B) est un agent anhydre ayant un pH de 1 à 6, de préférence de 1,3 à 4,5, plus préférentiellement de 1,6 à 4,0 et particulièrement préférentiellement de 2,0 à 3,6, et
- l'agent (c) dans le récipient (C) a un pH de 0,5 à 4,0, de préférence de 0,7 à 3,5, plus préférentiellement de 0,9 à 3,0 et particulièrement préférentiellement de 1, 1 à 2,5.

11. Unité de conditionnement multi-composante (*kit of parts*) selon une des revendications 1 à 10, **caractérisée en ce que**
- la quantité totale de tous les colorants et précurseurs de colorants d'oxydation contenue dans l'agent (a) est de 0,2 % en poids maximum, de préférence de 0,1 % en poids maximum, plus préférentiellement de 0,05 % en poids maximum et particulièrement préférentiellement de 0,01 % en poids maximum - rapportée au poids total de l'agent (a),
- la quantité totale de tous les colorants et précurseurs de colorants d'oxydation contenue dans l'agent (b) est de 0,2 % en poids maximum, de préférence de 0,1 % en poids maximum, plus préférentiellement de max. 0,05 % en poids et particulièrement préférentiellement de max. 0,01 % en poids - rapportée au poids total de l'agent (b), et
- la quantité totale de tous les colorants et précurseurs de colorants d'oxydation contenue dans l'agent (c) est de 0,2 % en poids maximum, de préférence de 0,1 % en poids maximum, plus préférentiellement de 0,05 % en poids maximum et particulièrement préférentiellement de 0,01 % en poids maximum - rapportée au poids total de l'agent (c).

12. Unité de conditionnement multi-composante (*kit of parts*) selon une des revendications 1 à 11, **caractérisée en ce que**
- la quantité totale de tous les oxydants du groupe des peroxydes et des persulfates contenus dans le produit (a) est de 0,2 % en poids maximum, de préférence de 0,1 % en poids maximum, plus préférentiellement de 0,05 % en poids maximum et particulièrement préférentiellement de 0,01 % en poids maximum - rapportée au poids total de l'agent (a),
- la quantité totale de tous les oxydants du groupe des peroxydes et des persulfates contenus dans le produit (a) est de. 0,2 % en poids maximum, de préférence de 0,1 % en poids maximum, plus préférentiellement de 0,05 % en poids maximum et particulièrement préférentiellement de 0,01 % en poids maximum - rapportée au poids total de l'agent (b), et
- la quantité totale de tous les oxydants du groupe des peroxydes et des persulfates contenus dans le produit (a) est de 0,2 % en poids maximum, de préférence de 0,1 % en poids maximum, plus préférentiellement de 0,05 % en poids maximum et particulièrement préférentiellement de 0,01 % en poids maximum - rapportée au poids total de l'agent (c).

13. Procédé de décoloration réductrice de fibres de kératine colorées, comprenant les étapes suivantes dans l'ordre indiqué :
(I) préparer un produit décolorant prêt à l'emploi par mélange d'un agent (a), comme défini aux revendications 1, 2, 10, 11 et/ou 12, avec un agent (b), comme défini aux revendications 1, 3, 10, 11 et/ou 12,
(II) appliquer le produit décolorant prêt à l'emploi sur les fibres de kératine,
(III) laisser agir le produit décolorant sur une période de 5 à 60 minutes, de préférence de 10 à 55 minutes, plus préférentiellement de 20 à 50 minutes et particulièrement préférentiellement de 30 à 45 minutes,
(IV) rincer le produit décolorant des fibres de kératine,
(V) appliquer un agent cosmétique aqueux (c) sur les fibres de kératine, l'agent (c) étant un produit comme défini aux revendications 1, 4, 5, 6, 7, 8, 9, 10, 11 et/ou 12,
(VI) laisser agir l'agent (c) sur une période de 30 secondes à 30 minutes, de préférence de 30 secondes à 20 minutes, plus préférentiellement de 30 secondes à 10 minutes et particulièrement préférentiellement de 30 secondes à 5 minutes, et
(VII) rincer l'agent (c) sur les fibres de kératine.

14. Procédé selon la revendication 13, **caractérisé en ce que**, après l'étape (VII), les étapes suivantes doivent être effectuées dans l'ordre indiqué
(VIII) appliquer un agent cosmétique aqueux (c) sur les fibres de kératine, l'agent (c) étant un produit comme défini aux revendications 1, 4, 5, 6, 7, 8, 9, 10, 11 et/ou 12,
(IX) laisser agir l'agent (c) sur une période de 30 secondes à 30 minutes, de préférence de 30 secondes à 20 minutes, plus préférentiellement de 30 secondes à 10 minutes et particulièrement préférentiellement de 30 secondes à 5 minutes, et
(X) rincer l'agent (c) des fibres de kératine.

15. Procédé selon une des revendications 13 à 14, **caractérisé en ce que** le produit décolorant prêt à l'emploi est appliqué sur des fibres de kératine, qui ont été colorées avec au moins un précurseur de colorant d'oxydation issu du groupe constitué de p-phénylènediamine, p-toluylènediamine, N,N-bis-(β-hydroxyéthyl)-p-phénylènediamine, 2-(β-hydroxyéthyl)-p-phénylènediamine et/ou 2-(méthoxyméthyl)-p-phénylènediamine.
